# EUROPEAN PATENT APPLICATION

(11) **EP 4 495 259 A1**
(43) Date of publication of application: **22.01.2025**
(21) Application number: 23186500.7
(22) Date of filing: 19.07.2023
(51) Int. Cl.: C12Q 1/6883

(54) **DIAGNOSTIC METHODS OF NEURODEVELOPMENTAL DISORDERS**

(71) Applicant: Stichting Terre- Nederlandse Rett Syndroom Fonds, 2631NG Nootdorp (NL)
(72) Inventor: BAHRAM SANGANI, Nasim, NOOTDORP (NL); EIJSSEN, Lars Maria Theo, NOOTDORP (NL); KOETSIER, Jarno Jean Joseph, NOOTDORP (NL); CURFS, Leopold Maria Gerard, NOOTDORP (NL); REUTELINGSPERGER, Christiaan Peter Maria, NOOTDORP (NL)
(74) Representative: Algemeen Octrooi- en Merkenbureau B.V.

(57) **Abstract**

The invention relates to an in vitro method for the diagnosis and/or prognosis of a neurodevelopmental disorder, in which disorder one or more of neurogenesis, neural migration, and synaptogenesis events is impaired in relation to the event in case of no neurodevelopmental disorder, preferably for the diagnosis and/or prognosis of Rett syndrome, wherein the method comprises the analysis of one or more cluster of miRNAs. The invention also discloses the application of the method to analyze the progression of the disorder and/or to monitor the efficacy of a therapy. Simplified kits are also disclosed for the carry out of the methods.

## Description

The present invention relates to the field of diagnostic methods of neurodevelopmental disorders.

### BACKGROUND OF THE INVENTION

A neurodevelopmental disorder occurs when impairments of the growth and development of the brain and/or central nervous system takes place for any reason. Examples of neurodevelopmental disorders include the neurogenic disorders, such as Fragile X syndrome, Down syndrome, Rett syndrome, and hypogonadotropic hypogonadal syndromes.

The development of the nervous system is complex and involves an accurate spatiotemporal regulation influenced by genetic programs and by the environment. Any significant deviation from the normal developmental trajectory results in an altered or missing functional neuronal connectivity, structure and architecture. The neurogenesis, neural migration, and synaptogenesis during early nervous system development, in particular of the brain, are key aspects for a normal functionality. The causes of neurodevelopmental disorders are diverse, and they may affect different areas of the nervous system at different times and ages. A subgroup of these disorders are due to genetic causes, known as neurogenic disorder multifactorial syndromes, which have many causes that converge to a more specific neurodevelopmental manifestation. Among these, the Rett syndrome is included.

Rett syndrome is a rare genetic neurological and developmental disorder that affects the way the brain develops. This disorder causes a progressive loss of motor skills and language. Loss-of-function spontaneous mutations in the X-linked gene Methyl CpG Binding Protein 2 (MECP2) are identified as the cause of classical Rett syndrome (RTT) in more than 95% of patients diagnosed worldwide, almost exclusively females, since males with the mutations die shortly after birth or in the uterus. RTT is a neurological disorder characterized by the onset of progressive deterioration of cognitive and motor skills after 6-18 months of age. However, the underlying molecular alterations can arise as early as upon neural maturation during prenatal brain development. Rett syndrome is a disabling disease: although some girls may retain some control over their hand movements and ability to walk and communicate, the majority become completely dependent on care, which they require 24 hours. This leads to a significant healthcare burden. Conditions such as arrhythmia can significantly shorten lifespan, but many women do survive into middle age and older. Current diagnosis is based on the symptoms and can be confirmed with genetic testing. There is no particular cure for the disorder, and the treatment is focused on improving symptoms. Anticonvulsants may be used to help with seizures. Special education and physiotherapy is also accommodated depending on the needs of the child. Many of those with the condition live into middle age. In 2023, the Food and Drug Administration (FDA) has approved trofinetide (Daybue (https://acadia.com/our-medicines/daybue/ ); Acadia Pharmaceuticals, San Diego, CA) for the treatment of Rett syndrome in adult and pediatric patients 2 years of age and older. This drug (a synthetic analog of insulin-like growth factor 1 (IGF-1) peptide) represents the first and only approved therapy for the rare genetic neurodevelopmental disorder.

The early diagnosis and the progression of the Rett syndrome, and of other neurodevelopmental disorders in which neurogenesis, synaptogenesis or neural migration are in some way compromised, are of high importance. Unfortunately, current diagnostic methods do not allow an early diagnosis and/or the study of the progression of the disorders, which in most of the times compromises the proper and fast selection of any medical intervention.

Moreover, mainly due to the inaccessibility of living human brain tissue, is nowadays difficult to have a good model for these disorders, which could help to elucidate the alterations, for example mutation-induced, in neurogenesis, neural migration, and synaptogenesis. This could, from the one side, help to understand the underlying events in these disorders, and on the other hand to provide insights to detectable signals (i.e., biomarkers) of these alterations.

### SUMMARY OF THE INVENTION

The authors of the present invention have surprisingly found a strong and specific correlation between miRNA in extracellular vesicles (EVs) and neurodegenerative and neurological defective processes that take place in disorders with neurodevelopmental impaired events.

The data derive from a cell model, i.e. a forebrain organoid, that fully resembles the brain of subjects with Rett syndrome during central nervous system development, but that it is equally applicable to other disorders in which neurogenesis, neural migration, and synaptogenesis are impaired or altered. The model allows the comparison of these neurodevelopmental events between a disorder condition and a non-disorder condition.

The analysis of certain miRNA clusters provides the information at an early stage of any disorder associated with the neural development, which is critical and indeed the reason of the observed symptomatology in these disorders. The differential expression of the miRNAs are informative of impaired or altered neurogenesis, synaptogenesis and neural migration in these disorders, preferably of Rett syndrome.

Thus, a first aspect of the invention relates to an in vitro method for the diagnosis and/or prognosis of a neurodevelopmental disorder, in which one or more of neurogenesis, neural migration, and synaptogenesis events is impaired in relation to the absence of neurodevelopmental disorder (i.e., in relation to the event in case of no neurodevelopmental disorder), wherein the method comprises:
(a) isolating extracellular vesicles (EV) from an isolated sample of a subject;
(b) collecting miRNA from the EVs;
(c) determining the expression level of at least one or more of a miRNA of the miR-302/367 cluster and/or at least one or more of a miRNA of the chromosome 14 miRNA cluster (C14MC); and
(d) comparing the said expression levels to one or several reference expression levels or range of reference expression levels,

wherein the subject is diagnosed of a neurodevelopmental disorder, preferably of Rett syndrome, when the expression of at least one of the miRNA of the miR-302/367 cluster and/or of at least one of the miRNA of C14MC, are equal to a reference expression level of a subject suffering from the neurodevelopmental disorder, preferably from Rett syndrome, for that miRNA(s) of the respective clusters(s), or
wherein the subject is diagnosed of not suffering a neurodevelopmental disorder, preferably not suffering of Rett syndrome, when the expression level of the determined miRNA of the miR-302/367 and/or C14MC clusters are not equal to the reference expression level of a subject suffering from the neurodevelopmental disorder, preferably of a subject suffering from Rett syndrome,
being all miRNA clusters defined according to the miRBase Release 22.1 of the miRBase.

Also another aspect of the invention is an in vitro method for the analysis of progression of a neurodevelopmental disorder and/or for the monitoring of a treatment of a subject previously diagnosed of a neurodevelopmental disorder, in which disorder one or more of neurogenesis, neural migration, and synaptogenesis events is impaired in relation to the event in case of no neurodevelopmental disorder, preferably the method for the analysis of the progression and/or for the monitoring of a treatment of a subject previously diagnosed of Rett syndrome, wherein the expression level of one or more of a miRNA of the miR-302/367 cluster and/or of one or more of a miRNA of the chromosome 14 miRNA cluster (C14MC) are determined at two or more time points, and the progression of the disorder and/or the effect of a monitored treatment is determined by evaluating the relative variation of the expression level of the one or more of the determined miRNA(s) of the miR-302/367 and/or C14MC clusters within the two or more time points.

As will be illustrated in the examples below, these methods provide for an easy analysis of the disorders in their early stages. The data from the organoid model used are transferable to the actual condition. It is widely accepted that organoid technologies equip the scientist community with the tools to investigate mechanisms of brain diseases and novel disease phenotypes, which eventually lead to better diagnostic methods and therapies (see Sidhaye et al. 2021. Brain organoids: an ensemble of bioassays to investigate human neurodevelopment and disease. Cell Death & Differentiation. 28:52-67 https://doi.org/10.1038/s41418-020-0566-4).

MicroRNA (miRNA) clusters are defined by a set of two or more miRNAs, which are transcribed from physically adjacent miRNA genes. MiRNA genes can be found either in protein-coding or non-coding regions of transcription units (TUs). This miR-302/367 cluster is located on human chromosome 4 within the intron of the long non-coding gene MIR302CHG and the coding gene LARP7 in the sense and antisense direction, respectively. Genetic variants in these genes have been associated with Alazami Syndrome, a disorder characterized by growth restriction and intellectual disability, suggesting an important role of this genomic region in (neural) development. The hsa-miR-302/367 cluster has also previously been involved in the control of ectodermal differentiation.

C14MC is one of the largest clusters of miRNAs in the human genome located at the imprinted DLK1-DIO3 domain on the long arm of chromosome 14 (14q32). C14MC has been considered as a pregnancy-related cluster with a crucial role in placental development.

These clusters miRNAs are implicated in the regulation of various cellular processes, including proliferation, differentiation, and apoptosis. Their dysregulations have also been reported in various cancer types as well as neurological disorders.

This is the first time these clusters have been correlated with the provision of information regarding neurodevelopment, and in particular correlation with Rett syndrome.

The in vitro method supposes, in addition, the provision for the first time of a tool to track the progression of any neurodevelopmental disorder, in particular of the Rett syndrome, which helps to see how a particular therapeutical approach or regimen is performing, at the same time it allows to alter any medical approach in time to improve the quality of life of the subjects. Therefore, the in vitro method of the invention can also be framed as a companion diagnostic method.

From all this also derives, as a further aspect of the invention, a method of deciding or recommending to initiate or to continue a therapy for a subject suspected of suffering from a neurodevelopmental disorder, in which disorder one or more of neurogenesis, neural migration, and synaptogenesis events is impaired in relation to the event in case of no neurodevelopmental disorder, preferably for a subject suffering from Rett syndrome, which method comprises the steps of:
(a) carrying out the diagnostic method as defined in any one of the previous aspects;
(b) optionally determining the outcome of a physical motor movement inspection, and/or cardiac function, and/or respiratory function, and/or swallowing function; and
(c) if the subject is diagnosed of suffering from a neurodevelopmental disorder, preferably of suffering from Rett syndrome, and optionally of the stage of the disorder being determined, then initiation or continue of a treatment is recommended.

For the carrying out of the methods of the invention disclosed in the previous aspects, particular kits comprising the means for determining the expression level of one of the miRNAs are used. Therefore, yet another aspect of the invention is the use of a kit comprising means for determining the expression level of one of the miRNA of the miR-302/367 cluster and/or the expression level of at least one of the miRNA of C14MC, and optionally means for collecting miRNA from EVs, for the diagnosis and/or prognosis of a neurodevelopmental disorder, preferably for the diagnosis and/or prognosis of Rett syndrome.

With the aim of simplifying the diagnosis and/or prognosis at ambulatory level or at the moment of birth, the invention also provides, as another aspect, simplified kits comprising means only for the detection of the levels of expression of at least one of the miRNA of the miR-302/367 cluster, and/or of at least one of the miRNAs of C14MC. This aspect can also be formulated as a kit comprising means for determining the expression level of at least one of the miRNA of the miR-302/367 cluster and/or of at least one of the miRNAs of C14MC, and optionally means for collecting miRNA from EVs, wherein the means are at least 10% of the reagents in the kit for assaying miRNA.

According to the best knowledge of the inventors, this is the first time the clusters miR-302/367 and C14MC have been associated with the capability to diagnose, prognose or indicate efficacy of a therapy in a neurodevelopmental disorder, in particular in Rett syndrome.

Thus, is also another aspect of the invention the use of the expression level in extracellular vesicles (EVs) of at least one or more of a miRNA of the miR-302/367 cluster and/or at one or more of a miRNA of the chromosome 14 miRNA cluster, as marker of clinical diagnosis and/or prognosis of a neurodevelopmental disorder, preferably of Rett syndrome, and/or as progression of disorder or of efficacy of therapy.

It is another aspect of the invention to provide an algorithm for carrying out any of the methods of diagnosis and/or of prognosis as defined in the above aspects. In the sense of the invention, the term "algorithm" is also synonymous of panel or decision diagrams, predictors and combinatory of data to correctly categorize an individual sample. According to aspects and embodiments of the invention, diagnosis and/or prognosis of a neurodevelopmental disorder can be performed using a mathematical algorithm that assesses a detectable level of miRNA, comprising one or more of the miRNA of the clusters for the diagnosis and/or prognosis of the neurodevelopmental disorders as described above, either in conjunction with or independent of other clinical parameters, to correctly categorize an individual sample as originating from a subject suffering from a disorder, a subject not suffering from the disorder, or, as described above, to further categorize an individual sample as originating from a subject with a particular stage of the disorder (i.e., progression), or as originating from a subject in which a certain applied therapy resulted effective or not.

The present invention also encompasses an in vitro method for the diagnosis and/or prognosis of a neurodevelopmental disorder, in particular a brain neurodevelopmental disorder in which one or more of neurogenesis, neural migration, and synaptogenesis events is impaired, in relation to the event in case of no neurodevelopmental disorder, preferably for the diagnosis and/or prognosis of Rett syndrome, in which the expression level of one more miRNA of C14MC selected from the group consisting of hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p is determined; and/or_the expression levels of one or more miRNA of C14MC selected from the group consisting of hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p is determined, in an isolated sample of a subject, preferably comprising EVs, being all miRNA clusters defined according to the miRBase Release 22.1 of the miRBase, as retrievable on 06.06.2023.

### DRAWINGS

Embodiments of the invention are further described hereinafter with reference to the accompanying drawings, in which:

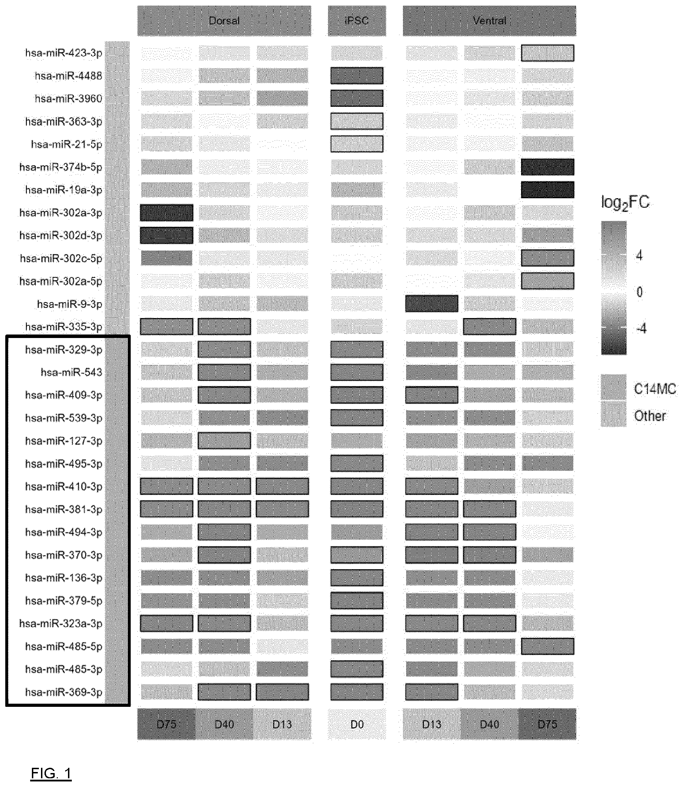

FIG. 1 is a heatmap of differentially expressed miRNAs. The logarithm of fold change (log2FC) per time point (D0, D>>>) and region (Dorsal or Ventral) are shown for the miRNAs with a significantly different expression between Rett syndrome (RTT, MeCP2:R255X) and isogenic controls (IC) in at least one time point. The black border indicates statistical significance (i.e., FDR-adjusted p-value < 0.05). The boxed miRNAs on the left indicate that the corresponding miRNAs are part of the chromosome 14 miRNA cluster (C14MC).

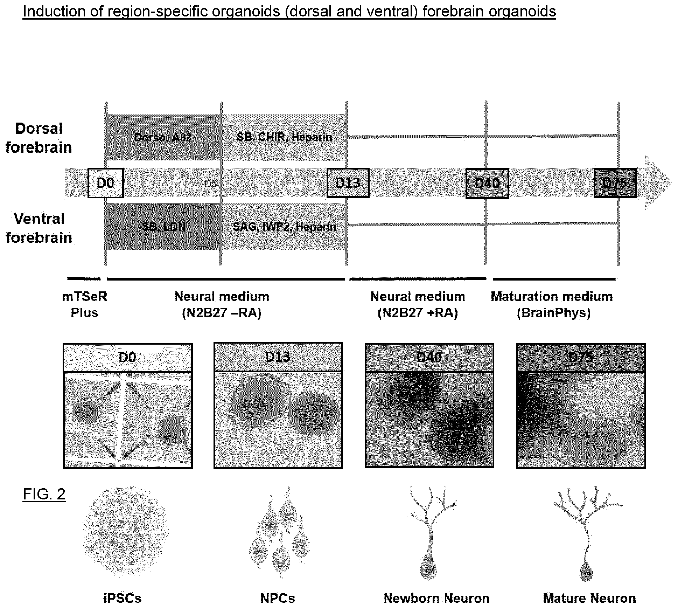

FIG. 2 illustrates an overview of the applied methodology to develop the forebrain organoids. Extracellular vesicle miRNA and protein expression data from dorsal and ventral forebrain organoids were collected at four time points, corresponding to the different developmental stages. At day 0 (D0) human iPSCs are seeded in microwell plates to form aggregates. At day 13 (D13) neuronal progenitor cells (NPCs) as well as neural rosettes appear. At day 40 (D40) of differentiation, newborn neurons of deep cortical layers VI and V emerge. Finally, at day 75 (D75) neurons are mature and fully functional. At this stage, dorsal forebrain cells resemble glutamatergic (excitatory) neurons while cells from the ventral forebrain mimic GABAergic (inhibitory) neurons. Brain organoids also contain astrocytes and glial cells, however, they are not schematically represented here. Schematic visualization was created using BioRender.com.

### DETAILED DESCRIPTION OF THE INVENTION

A portion of this disclosure contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

For purposes of the present invention, the following terms are defined below.

As used herein, the singular forms "a," "an" and "the" include plural referents unless the context clearly dictates otherwise. The indefinite articles "a" and "an" are synonymous with "at least one" or "one or more".

As used herein, the term "and/or" indicates that one or more of the stated cases may occur, alone or in combination with at least one of the stated cases, up to with all of the stated cases.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. The term "one or more" includes 1 and also 2, 3, 4, 5, 6, 7, 8, 9, 10, 11, 12, 13, 14, 15, ..., etc.

As used herein, "comprising" or "to comprise" is construed as being inclusive and open ended, and not exclusive. Specifically, the term and variations thereof mean the specified features, steps or components are included. These terms are not to be interpreted to exclude the presence of other features, steps or components. It also encompasses the more limiting "to consist of".

As used herein, "conventional techniques" or "methods known to the skilled person" refer to a situation wherein the methods of carrying out the conventional techniques used in methods as disclosed herein will be evident to the skilled worker. The practice of conventional techniques in molecular biology, biochemistry, cell culture, genomics, sequencing, medical treatment, pharmacology, immunology and related fields are well-known to those of skill in the art and are discussed, in various handbooks and literature references.

As used herein, the term "subject" refers to a mammal, for example a human, in particular a female human. It includes the term "patient".

As used herein, the terms "treatment" and "treating" refer to therapeutic treatment. The object of the treatment is to at least slow down the disease condition. Those in need of the treatment include those already with the disease condition.

The term "reference expression level" or "reference expression value', used interchangeably and as used herein, relates to a predetermined criteria used as a reference for evaluating the values/levels or data obtained from the samples collected from a subject. The reference value or reference level can be an absolute value; a relative value; a value that has an upper or a lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. Range of values of each miRNA and particular combinations of the values of the different miRNA provide for correct classification of subjects with high sensitivity and specificity.

The term "diagnosis" is known to the person skilled in the art. As used herein "diagnosis" is understood as becoming aware of a particular medical condition, complication or risk in a subject; the determination of the nature of the disease or condition; or the distinguishing of one disease or condition from another (i.e., differential diagnosis). It refers both to the process of attempting to determine or identify the possible disease or disorder, and to the opinion reached by this process. A diagnosis, in the sense of diagnostic procedure, can be regarded as an attempt at classification of an individual's condition into separate and distinct categories that allow medical decisions about treatment and prognosis to be made.

Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification.

"Prognosis" as used herein refers to the prediction of the probable progression and outcome of a disease.

"Progression" refers to the determination of the evolution to a differentiated stage of a particular disorder, which can be a worsening or an improvement of the associated symptomatology and/or of the physiologic parameters therein involved.

The term "stage of disorder" relates to the or level of severity of a disease. In the particular case of Rett syndrome the four stages are stage I, called early-onset; stage II, the rapid destructive stage; stage III, the plateau or pseudo-stationary stage; and stage IV, the late motor deterioration stage.

"Neurogenesis" refers to the process by which nervous system cells, the neurons, are produced by neural stem cells (NSCs). Detection of neurogenesis is commonly achieved by immunohistochemical detection of newly generated cells labeled with the thymidine analog bromodeoxyuridine (BrdU), assessed with their coexpression of mature neuronal markers, such as neuronal nuclei (NeuN), or mature glial markers, such as S100β for mature astrocytes. These and other commonly used technologies are widely known by the skilled person in the art.

"Neural or neuronal migration" is the method by which neurons travel from their origin or birthplace to their final position in the brain. A number of molecular techniques widely used in the field of studying neuronal migration include cell cycle analysis, morphology analysis, and migratory analysis of migration of newly born neuronal cells. The skilled person will be aware of all these technologies.

"Synaptogenesis" is the process by which synapses between neurons in the nervous system are formed. Synaptogenesis is particularly important during an individual's critical period, during which there is a certain degree of synaptic pruning due to competition for neural growth factors by neurons and synapses. Processes that are not used, or inhibited during their critical period will fail to develop normally later on in life. A number of methods have been used to measure synaptogenesis in vitro, including quantification of synaptic protein levels using antibody-based methods (e.g., ELISA, western blotting and immunocytochemistry) and functional assessment using electrophysiology, or high-content imaging. The skilled person will be aware of all these technologies.

A "miRNA" is a naturally occurring, small non-coding RNA that is about 17 to about 25 nucleotide (nt) in length in its biologically active form that negatively regulates mRNA translation on a sequence-specific manner. Identified miRNAs are registered in the miRNA database miRBase (miRBase: the microRNA database. Release 22.1, as retrievable on 6 of June 2023, https://www.mirbase.org/index.shtml)

The present invention refers to miRNAs unequivocally identified in the miRBase Release 22.1 of the miRBase. The listed miRNA correspond to the human genome, but the skilled person in the art will understand or know that homologues of other than human are also included, in particular homologues of the human miRNAs in other mammals in which neurodevelopmental disorders, in particular brain neurodevelopmental disorders, have also been recognized and that in many occasions have been used as animal models of the disorder in humans.

"Extracellular vesicles (EVs)" are lipid bilayer-delimited particles that are naturally released from almost all types of cells but, unlike a cell, cannot replicate. EVs range in diameter from near the size of the smallest physically possible unilamellar liposome (around 20-30 nanometers) to as large as 10 microns or more, although the vast majority of EVs are smaller than 200 nm. EVs can be divided according to size and synthesis route into exosomes, microvesicles and apoptotic bodies. They carry a cargo of proteins, nucleic acids, lipids, metabolites, and even organelles from the parent cell. The skilled person in the art knows the conventional techniques for their isolation from an isolated sample of a subject.

As previously indicated, a first aspect of the invention is an in vitro method for the diagnosis and/or prognosis of a neurodevelopmental disorder, in which disorder one or more of neurogenesis, neural migration, and synaptogenesis events is impaired in relation to the absence of neurodevelopmental disorder, wherein the method comprises:
(a) isolating extracellular vesicles (EV) from a sample isolated from a subject;
(b) collecting miRNA from the EVs;
(c) determining the expression level of at least one or more of a miRNA of the miR-302/367 cluster and/or at least one or more of a miRNA of the chromosome 14 miRNA cluster (C14MC); and
(d) comparing the said expression levels to one or several reference expression levels or ranges of reference expression levels,

wherein the subject is diagnosed of a neurodevelopmental disorder, preferably of Rett syndrome, when the expression of at least one of the miRNA of the miR-302/367 cluster, and/or of at least one of the miRNA of C14MC, are equal to or similar (i.e., within a margin considered equivalent) to a reference expression level of a subject suffering from the neurodevelopmental disorder, preferably suffering from Rett syndrome, for that miRNA of the respective clusters, or
wherein the subject is diagnosed of not suffering a neurodevelopmental disorder, preferably of not suffering from Rett syndrome, when the expression level of the determined miRNA of the miR-302/367 and/or C14MC clusters are not equal to the expression level of a subject suffering from the neurodevelopmental disorder, preferably suffering from Rett syndrome, being all miRNA clusters defined according to the miRBase Release 22.1 of the miRBase.

The expression `in relation to absence of the disorder' means that any of the neurogenesis, neural migration, and synaptogenesis events are within the accepted as in condition of no disorder. The expression can also be paraphrased as `in relation to the event in case of no neurodevelopmental disorder'.

In a particular embodiment of the in vitro method for the diagnosis and/or prognosis of a neurodevelopmental disorder, the method is for the analysis of progression of disease and/or for the monitoring of a treatment of a subject previously diagnosed of a neurodevelopmental disorder, preferably Rett syndrome. In a more particular embodiment of the method for the analysis of progression of disease and/or for the monitoring of a treatment, preferably the expression level of one or more of a miRNA of the miR-302/367 cluster and/or at least of one or more of a miRNA of the chromosome 14 miRNA cluster (C14MC) are determined at two or more time points, and the progression and/or the effect of a monitored treatment is determined by evaluating the relative variation of the expression level of the one or more of the determined miRNA of the miR-302/367 and/or C14MC clusters within the two or more time points. Particular two or more time points include time points at different days, preferably with at least one day in-between; time points at different months; and time points at different years. The relative variation of the expression level within the two or more time points comprises either the increase or decrease of the levels of expression of the one or more of the determined miRNA. In particular, it comprises the relative variation of any ratio or score obtained with the processing of levels at the two time points.

In a more particular embodiment of the method for the analysis of progression of disease, and/or for the monitoring of a treatment of a subject previously diagnosed of a neurodevelopmental disorder and receiving an initial therapy, (i) if the relative variation within the two or more time points of the expression level of one or more of a miRNA of the miR-302/367 cluster and/or of one or more of a miRNA of the chromosome 14 miRNA cluster (C14MC) is indicative of progression of disease and/or of non-effectivity of a monitored treatment, the patient is selected for a different therapy, and (ii) if this relative variation within the two or more time points is indicative of no progression of disease and/or of effectivity of a monitored treatment, the patient is selected for a therapy selected from the continuing of the initial therapy, and an additional or alternative therapy.

In another particular embodiment of the in vitro method disclosed in the above aspects and embodiments, the step (b) of collecting miRNA from the EVs is carried out in a buffered solution. The skilled person in the art will be aware or know of the buffered solutions adequate for the collection and preservation of miRNAs which levels are to be determined later.

In another particular embodiment, the in vitro method disclosed in the above aspects and embodiments is one wherein: step (c) comprises determining the expression level of a set of miRNA that comprises at least one or more of a miRNA of the miR-302/367 cluster and at least one or more of a miRNA of the chromosome 14 miRNA cluster (C14MC); and step (d) comprises comparing the said expression levels to one or several reference expression levels or range of reference expression levels.

In this particular embodiment that comprises determining the expression level of a set of miRNA that comprises at least one or more of a miRNA of the miR-302/367 cluster and at least one or more of a miRNA of the chromosome 14 miRNA cluster (C14MC):
- the subject is diagnosed of a neurodevelopmental disorder, preferably of Rett syndrome, when the expression of at least one of the miRNA of the miR-302/367 cluster and of at least one of the miRNA of C14MC are equal to or similar to a reference expression level of a subject suffering from the neurodevelopmental disorder, preferably equal to a reference expression level of a subject suffering from Rett syndrome, for that miRNA(s) of the respective cluster(s); or
- the subject is diagnosed of not suffering a neurodevelopmental disorder, preferably not suffering from Rett syndrome, when the expression level of the determined miRNA of the miR-302/367 and C14MC clusters are not equal to the expression level of a subject suffering from the neurodevelopmental disorder, preferably not equal to the expression level of a subject suffering from Rett syndrome.

Also, or alternatively, in this embodiment of the method that comprises determining the expression level of a set of miRNA that comprises at least one or more of a miRNA of the miR-302/367 cluster, and at least one or more of a miRNA of the chromosome 14 miRNA cluster (C14MC), preferably the expression level of one or more of a miRNA of the miR-302/367 cluster and at least of one or more of a miRNA of the chromosome 14 miRNA cluster (C14MC) are determined at two or more time points, and the progression of the neurodevelopmental disorder, in particular of Rett syndrome, and/or the effect of a monitored treatment, is (are) determined by evaluating the relative variation of the expression level of the one or more of the determined miRNA of the miR-302/367 and C14MC clusters within the two or more time points

In yet another particular embodiment of the in vitro method of the first aspect, the one or more miRNA of the miR-302/367 cluster is selected from the group consisting of hsa-miR-302a-3p, hsa-miR-302d-3p, hsa-miR-302c-5p and hsa-miR-302a-5p.

The miR-302/367 cluster includes ten mature miRNA sequences.

The microRNA (miRNA) hsa-miR-302a is described by accession number MI0000738 in miRbase and may further be referred to as MIR302A, which is the full length hairpin also listed as SEQ ID NO: 94 (302ahp_ CCACCACTTAAACGTGGATGTACTTGCTTTGAAACTAAAGAAGTAAGTGCTTCCATGT TTTGGTGATGG). From the hairpin the mature sequence hsa-miR-302a-3p is derived, which is described by accession number MIMAT0000684 in miRbase and described herein as SEQ ID NO: 1 (TAAGTGCTTCCATGTTTTGGTGA). From the hairpin the mature sequence hsa-miR-302a-5p is derived, which is described by accession number MIMAT0000683 in miRbase and described herein as SEQ ID NO: 2 (TAAACGTGGATGTACTTGCTTT).

The microRNA (miRNA) hsa-miR-302d is described by accession number MI0000774 in miRbase and may further be referred to as MIR302D, which is the full length hairpin also listed as SEQ ID NO: 95 (302dhp_ CCTCTACTTTAACATGGAGGCACTTGCTGTGACATGACAAAAATAAGTGCTTCCATGT TTGAGTGTGG). From the hairpin the mature sequence hsa-miR-302d-5p is derived, which is described by accession number MIMAT0004685 in miRbase and described herein as SEQ ID NO: 3 (ACTTTAACATGGAGGCACTTG). From the hairpin the mature sequence hsa-miR-302d-3p is derived, which is described by accession number MIMAT0000718 in miRbase and described herein as SEQ ID NO: 4 (TAAGTGCTTCCATGTTTGAGTGT).

The microRNA (miRNA) hsa-miR-302c is described by accession number MI0000773 in miRbase and may further be referred to as MIR302C, which is the full length hairpin also listed as SEQ ID NO: 96 (302chp_ CCTTTGCTTTAACATGGGGGTACCTGCTGTGTGAAACAAAAGTAAGTGCTTCCATGTT TCAGTGGAGG). From the hairpin the mature sequence hsa-miR-302c-5p is derived, which is described by accession number MIMAT0000716 in miRbase and described herein as SEQ ID NO: 5 (TTTAACATGGGGGTACCTGCT). From the hairpin the mature sequence hsa-miR-302c-3p is derived, which is described by accession number MIMAT0000717 in miRbase and described herein as SEQ ID NO: 6 (AAGTGCTTCCATGTTTCAGTGGT).

The microRNA (miRNA) hsa-miR-302b is described by accession number MI0000772 in miRbase and may further be referred to as MIR302B, which is the full length hairpin also listed as SEQ ID NO: 97 (302bhp_ GCTCCCTTCAACTTTAACATGGAAGTGCTTTCTGTGACTTTAAAAGTAAGTGCTTCCAT GTTTTAGTAGGAGT). From the hairpin the mature sequence hsa-miR-302b-5p is derived, which is described by accession number MIMAT0000714 in miRbase and described herein as SEQ ID NO: 7 (ACTTTAACATGGAAGTGCTTTCT). From the hairpin the mature sequence hsa-miR-302b-3p is derived, which is described by accession number MIMAT0000715 in miRbase and described herein as SEQ ID NO: 8 (TAAGTGCTTCCATGTTTTAGTAG).

The microRNA (miRNA) hsa-miR-367 is described by accession number MI0000775 in miRbase and may further be referred to as MIR367, which is the full length hairpin also listed as SEQ ID NO: 98 (367hp_ CCATTACTGTTGCTAATATGCAACTCTGTTGAATATAAATTGGAATTGCACTTTAGCAA TGGTGATGG). From the hairpin the mature sequence hsa-miR-367-5p is derived, which is described by accession number MIMAT0004686 in miRbase and described herein as SEQ ID NO: 9 (ACTGTTGCTAATATGCAACTCTGTT). From the hairpin the mature sequence hsa-miR-367-3p is derived, which is described by accession number MIMAT0000719 in miRbase and described herein as SEQ ID NO: 10 (ATTGCACTTTAGCAATGGTGA).

In yet another particular embodiment of the in vitro method of the first aspect, optionally in combination with any of the embodiments above or below, the one more miRNA of C14MC is selected from the group consisting of hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p.

The mature sequences within this cluster are listed below. All sequences can be retrieved from miRBase Release 22.1, retrievable on 6 June 2023. Sequences in **bold** are significatively differentially expressed between RTT and isogenic controls (i.e., non-RTT condition)

| SEQ ID NO: X | *Name (accession number full length hairpin* / *mature sequence of miRBase release 22.1)* | Sequence |
|---|---|---|
| 11 | hsa-miR-127-5p (MI0000472 / MIMAT0004604) | CTGAAGCTCAGAGGGCTCTGATT |
| **12** | **hsa-miR-127-3p** (MI0000472 / MIMAT0000446) | **TCGGATCCGTCTGAGCTTGGCT** |
| 13 | hsa-miR-134-5p (MI0000474 / MIMAT0000447) | TGTGACTGGTTGACCAGAGGGG |
| 14 | hsa-miR-136-5p (MI0000475 / MIMAT0000448) | ACTCCATTTGTTTTGATGATGG |
| **15** | **hsa-miR-136-3p** (MI0000475 / MIMAT0004606) | **CATCATCGTCTCAAATGAGTCT** |
| 16 | hsa-miR-154-5p (MI0000480 / MIMAT0000452) | TAGGTTATCCGTGTTGCCTTC |
| 17 | hsa-miR-154-3p (MI0000480 / MIMAT0000453) | AATCATACACGGTTGACCTATT |
| 18 | hsa-miR-299-5p (MIMAT0002890) | TGGTTTACCGTCCCACATACAT |
| 19 | hsa-miR-299-3p (MIMAT0000687) | TATGTGGGATGGTAAACCGCTT |
| 20 | hsa-miR-323b-3p (MI0014206 / MIMAT0015050) | CCCAATACACGGTCGACCTCT |
| 21 | hsa-miR-323a-5p (MI0000807 / MIMAT0004696) | AGGTGGTCCGTGGCGCGTTCG |
| **22** | **hsa-miR-323a-3p** (MI0000807 / MIMAT0000755) | **GCACATTACACGGTCGACCTCT** |
| 23 | hsa-miR-329-5p (MI0001725 / MIMAT0026555) | AGAGGTTTTCTGGGTCTCTGTT |
| **24** | **hsa-miR-329-3p** (MI0001725 / MIMAT0001629) | **AACACACCTGGTTAACCTCTTT** |
| 25 | hsa-miR-337-5p (MI0000806 / MIMAT0004695) | GAACGGCTTCATACAGGAG |
| 26 | hsa-miR-337-3p (MI0000806 / MIMAT0000754) | CTCCTATATGATGCCTTTCTTC |
| 27 | hsa-miR-369-5p (MI0000777 / MIMAT0001621) | AGATCGACCGTGTTATATTCG |
| **28** | **hsa-miR-369-3p** (MI0000777 / MIMAT0000721) | **AATAATACATGGTTGATCTTT** |
| 29 | hsa-miR-370-5p (MI0000778 / MIMAT0026483) | CAGGTCACGTCTCTGCAGTTAC |
| **30** | **hsa-miR-370-3p** (MI0000778 / MIMAT0000722) | **GCCTGCTGGGGTGGAACCTGGT** |
| 31 | hsa-miR-376c-5p (MI0000776 / MIMAT0022861) | TGGATATTCCTTCTATGTTTA |
| 32 | hsa-miR-376c-3p (MI0000776 / MIMAT0000720) | AACATAGAGGAAATTCCACGT |
| 33 | hsa-miR-376b-5p (MI0002466 / MIMAT0022923) | TGGATATTCCTTCTATGTTTA |
| 34 | hsa-miR-376b-3p (MI0002466 / MIMAT0002172) | ATCATAGAGGAAAATCCATGT |
| 35 | hsa-miR-376a-2-5p (MI0003529 / MIMAT0022928) | GTGGATTTTCCTTCTATGGTTA |
| 36 | hsa-miR-376a-3p (MI0000784 / MIMAT0000729) | ATCATAGAGGAAAATCCACGT |
| 37 | hsa-miR-376a-5p (MI0000784 / MIMAT0003386) | GGTAGATTCTCCTTCTATGAGTA |
| 38 | hsa-miR-377-5p (MI0000785 / MIMAT0004689) | AGAGGTTGCCCTTGGTGAATTC |
| 39 | hsa-miR-377-3p (MI0000785 / MIMAT0000730) | ATCACACAAAGGCAACTTTTGT |
| **40** | **hsa-miR-379-5p** (MI0000787 / MIMAT0000733) | **TGGTAGACTATGGAACGTAGG** |
| 41 | hsa-miR-379-3p (MI0000787 / MIMAT0004690) | TATGTAACATGGTCCACTAAC |
| 42 | hsa-miR-380-5p (MI0000788 / MIMAT0000734) | ATGGTTGACCATAGAACATGCG |
| 43 | hsa-miR-380-3p (MI0000788 / MIMAT0000735) | TATGTAATATGGTCCACATCT |
| 44 | hsa-miR-381-5p (MI0000789 / MIMAT0022862) | AGCGAGGTTGCCCTTTGTATA |
| **45** | **hsa-miR-381-3p** (MI0000789 /MIMAT0000736) | **TATACAAGGGCAAGCTCTCTGT** |
| 46 | hsa-miR-382-5p (MI0000790 / MIMAT0000737) | GAAGTTGTTCGTGGTGGATTCG |
| 47 | hsa-miR-382-3p (MI0000790 / MIMAT0022697) | AATCATTCACGGACAACACTTT |
| 48 | hsa-miR-409-5p (MI0001735 / MIMAT0001638) | AGGTTACCCGAGCAACTTTGCAT |
| **49** | **hsa-miR-409-3p** (MI0001735 / MIMAT0001639) | **GAATGTTGCTCGGTGAACCCCT** |
| **50** | **hsa-miR-410-3p** (MI0002465 / MIMAT0002171) | **AATATAACACAGATGGCCTGT** |
| 51 | hsa-miR-411-5p (MI0003675 / MIMAT0003329) | TAGTAGACCGTATAGCGTACG |
| 52 | hsa-miR-411-3p (MI0003675 / MIMAT0004813) | TATGTAACACGGTCCACTAAC |
| 53 | hsa-miR-412-5p (MI0002464 / MIMAT0026557) | TGGTCGACCAGTTGGAAAGTAAT |
| 54 | hsa-miR-412-3p (MI0002464 / MIMAT0002170) | GTACTTCACCTGGTCCACTAGC |
| 55 | hsa-miR-431-5p (MI0001721 / MIMAT0001625) | TGTCTTGCAGGCCGTCATGCA |
| 56 | hsa-miR-431-3p (MI0001721 / MIMAT0004757) | CAGGTCGTCTTGCAGGGCTTCT |
| 57 | hsa-miR-432-5p (MI0003133 / MIMAT0002814) | TCTTGGAGTAGGTCATTGGGT |
| 58 | hsa-mir-432-3p (MI0003133 / MIMAT0002815) | ATTTCCTTACGTGGGCCACTGGA |
| 59 | hsa-miR-433-5p (MI0001723 / MIMAT0026554) | TACGGTGAGCCTGTCATTATT |
| 60 | hsa-miR-433-3p (MI0001723 / MIMAT0001627) | ATCATGATGGGCTCCTCGGTGT |
| **61** | **hsa-miR-485-5p** (MI0002469/ MIMAT0002175) | **AGAGGCTGGCCGTGATGAATTCG** |
| **62** | **hsa-miR-485-3p** (MI0002469/ MIMAT0002176) | **GTCATACACGGCTCTCCTCTCT** |
| 63 | hsa-miR-487a-5p (MI0002471 / MIMAT0026559) | GTGGTTATCCCTGCTGTGTTCG |
| 64 | hsa-miR-487a-3p (MI0002471/ MIMAT0002178) | AATCATACAGGGACATCCAGTT |
| 65 | hsa-miR-487b-5p (MI0003530 / MIMAT0026614) | TGGTTATCCCTGTCCTGTTCG |
| 66 | hsa-miR-487b-3p (MI0003530 / MIMAT0003180) | AATCGTACAGGGTCATCCACTT |
| 67 | hsa-miR-493-5p (MI0003132 / MIMAT0002813) | TTGTACATGGTAGGCTTTCATT |
| 68 | hsa-miR-493-3p (MI0003132 / MIMAT0003161) | TGAAGGTCTACTGTGTGCCAGG |
| 69 | hsa-miR-494-5p (MI0003134 / MIMAT0026607) | AGGTTGTCCGTGTTGTCTTCTC |
| **70** | **hsa-miR-494-3p** (MI0003134 /MIMAT0002816) | **TGAAACATACACGGGAAACCTCT** |
| **71** | **hsa-miR-495-3p** (MI0003135/ MIMAT0002817) | **AAACAAACATGGTGCACTTCTT** |
| 72 | hsa-miR-496 (MIMAT0002818) | TGAGTATTACATGGCCAATCT |
| 73 | hsa-miR-539-5p (MI0003514 / MIMAT0003163) | GGAGAAATTATCCTTGGTGTGT |
| **74** | **hsa-miR-539-3p** (MI0003514 /MIMAT0022705) | **ATCATACAAGGACAATTTCTTT** |
| 75 | hsa-miR-541-5p (MI0005539 / MIMAT0004919) | AAGGATTCTGCTGTCGGTCCCACT |
| 76 | hsa-miR-541-3p (MI0005539/MIMAT0004920) | TGGTGGGCACAGAATCTGGACT |
| **77** | **hsa-miR-543 (MI0005565/ MIMAT0004954)** | **AAACATTCGCGGTGCACTTCTT** |
| 78 | hsa-miR-544a (MI0003515/MIMAT0003164) | ATTCTGCATTTTTAGCAAGTTT |
| 79 | hsa-miR-654-5p (MI0003676 / MIMAT0003330 | TGGTGGGCCGCAGAACATGTGC |
| 80 | hsa-miR-654-3p (MI0003676 /MIMAT0004814) | TATGTCTGCTGACCATCACC |
| 81 | hsa-miR-655-3p (MI0003677/ MIMAT0003331) | ATAATACATGGTTAACCTCTTT |
| 82 | hsa-miR-656-3p (MI0003678 / MIMAT0003332) | AATATTATACAGTCAACCTCT |
| 83 | hsa-miR-665 (MI0005563/ MIMAT0004952) | ACCAGGAGGCTGAGGCCC |
| 84 | hsa-mir-668-3p (MIMAT0003881) | TGTCACTCGGCTCGGCCCACTAC |
| 85 | hsa-miR-758-5p (MI0003757/ MIMAT0022929) | ATGGTTGACCAGAGAGCACACG |
| 86 | hsa-miR-758-3p (MI0003757/MIMAT0003879) | TTTGTGACCTGGTCCACTAAC |
| 87 | hsa-miR-770-5p (MI0005118/ MIMAT0003948) | TCCAGTACCACGTGTCAGGGCCAA |
| 88 | hsa-miR-889-3p ( MI0005540/ MIMAT0004921) | TTAATATCGGACAACCATTGT |
| 89 | hsa-miR-1185-5p ( MI0003821/ MIMAT0005798) | AGAGGATACCCTTTGTATGTTC |
| 90 | hsa-miR-1185-2-3p (MI0003821/ MIMAT0022713) | ATATACAGGGGGAGACTCT |
| 91 | hsa-miR-1185-1-3p (MI0003844/ MIMAT0022838) | ATATACAGGGGGAGACTCTTAT |
| 92 | hsa-miR-1197 (MI0006656 / MIMAT0005955) | TAGGACACATGGTCTACTTCT |
| 93 | hsa-miR-127-5p (MI0000472/ MIMAT0004604) | CTGAAGCTCAGAGGGCTCTGATT |

In all these polyribonucleotide sequences (RNA) the symbol "t" is construed as uracil, according to WIPO Standard ST.26.

In yet another particular embodiment of the in vitro method of the invention, the expression level of further one or more of hsa-miR-423-3p (MI0001445, MIMAT0001340), hsa-miR-4488 (MI0016849, MIMAT0019022), hsa-miR-3960 (MI0016964, MIMAT0019337), hsa-miR-363-3p (MI0000764, MIMAT0000707), hsa-miR-21-5p (MI0000077, MIMAT0000076), hsa-miR-374b-5p (MI0005566, MIMAT0004955), hsa-miR-19a-3p (MI0000073, MIMAT0000073), hsa-miR-9-3p (MI0000466, MIMAT0000442), and hsa-miR-335-3p (MI0000816, MIMAT0004703) is determined. The references into brackets correspond respectively to the entries of the full length hairpin / mature sequence in the miRBase database Release 22.1

More in particular the in vitro method of the invention comprises determining the following expression profile:
- in the miR-302/367 cluster, if one or more of hsa-miR-302a-3p and hsa-miR-302d-3p is down-expressed, and/or if one or more of hsa-miR-302c-5p and hsa-miR-302a-5p is up-expressed in relation to a subject not suffering a neurodevelopmental disorder, in particular not suffering from Rett syndrome; and
- in the C14MC, if one or more of the miRNA is up-expressed in relation to a subject not suffering neurodevelopmental disorder, in particular not suffering from Rett syndrome.

The term `expression profile', in this case miRNA expression profiling is defined in general as identifying the miRNAs expressed in a particular tissue, cell under a specified set of conditions and at a particular time, usually compared to expression in reference samples.

In even another particular embodiment of the in vitro method of the invention, in which step (c) comprises determining the expression level of a set of miRNA that comprises at least one or more of a miRNA of the miR-302/367 cluster, and at least one or more of a miRNA of the chromosome 14 miRNA cluster (C14MC), a ratio is established_between the expression level of the one or more miRNA determined within the C14MC and the expression level of the one or more miRNA determined within the miR-302/367 cluster. This ratio is a dimensionless value expressed optionally as a percentage and provides information about the presence of the disorders, the prognosis, progression or information of the efficacy of any applied therapy. In a more particular embodiment, the ratio embodiment the ratio consists in the expression level of the one or more miRNA determined within the C14MC divided by the expression level of the one or more miRNA determined within the miR-302/367 cluster.

In any of the previously disclosed aspects and corresponding embodiments of the in vitro method of the invention, the method comprise determining the expression levels of at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, or at least twenty miRNAs of the miR-302/367 cluster and C14MC together.

In even a more particular embodiment, the in vitro methods comprises determining the level of expression of all the following miRNAs of the miR-302/367 cluster: hsa-miR-302a-3p, hsa-miR-302d-3p, hsa-miR-302c-5p and hsa-miR-302a-5p.

In also another more particular embodiment, the in vitro methods comprises determining the level of expression of all the following miRNAs of the C14MC: hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p.

In even another more particular embodiment, the in vitro methods comprises determining the level of expression of the following miRNAs: hsa-miR-302a-3p, hsa-miR-302d-3p, hsa-miR-302c-5p and hsa-miR-302a-5p, hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p.

In another particular embodiment of the in vitro method according to any of the aspects and corresponding embodiments previously disclosed, the neurodevelopmental disorder is a brain neurodevelopmental disorder. In other particular embodiment, optionally in combination with the previous one, the neurodevelopmental disorder is a neurogenic disorder selected from Rett syndrome, Fragile X syndrome, Down syndrome, and hypogonadotropic hypogonadal syndromes, preferably Rett syndrome.

In another particular embodiment of the in vitro method of the fist aspect, the neurodevelopmental disorder is selected from the group consisting of: Autism spectrum disorder (ASD); Intellectual disabilities (IDs) or intellectual development disorder (IDD) and global developmental delay (GDD); Motor disorders including developmental coordination disorder, stereotypic movement disorder, and tic disorders (such as Tourette's syndrome), and CAS - Apraxia of speech; Neurogenetic disorders, in particular selected from Fragile X syndrome, Down syndrome, Rett syndrome, hypogonadotropic hypogonadal syndromes; Specific learning disorders, in particular dyslexia or dyscalculia; Traumatic brain injury, in particular congenital injuries that cause cerebral palsy, and disorders due to neurotoxicants; Fetal Alcohol Spectrum Disorders (FASD); and combination thereof.

During neurodevelopment, events such as the neurogenesis, differentiation of neural stem cells, development of neural circuits and plasticity are regulated by miRNAs. The examples in this description demonstrate that the hsa-miR-302/367 cluster and the C14MC detectable in EVs are involved in the normal and disorder-associated neurodevelopment, and that an altered expression is seen in case of disorder, which alteration correlates with an altered pattern of one or more of differentiation of neural stem cells, neural migration, synaptogenesis, and proliferation. All these altered neural cell-related events have been detected in Rett syndrome but also in other neurodevelopmental disorders.

In another particular embodiment of the in vitro methods according to the invention, optionally in combination with any of the embodiments above or below, the isolated sample of the subject is a body fluid, preferably selected from the group consisting of blood, plasma, serum, urine, sputum, and cerebrospinal fluid.

EVs are known to be able to cross the blood-brain barrier, therefore EV miRNAs, in particular miRNAs of the indicated clusters, associated to neurodevelopmental disorders, preferably associated to Rett syndrome, are blood-based biomarkers (i.e., blood, plasma, serum) for monitoring the progression of the disorder (e.g. Rett syndrome) after, for example, a specific intervention strategy.

Since the herewith proposed diagnostic method allows for the correct classification of a subject, the information retrieved from the same can be used in the taking of decisions for the clinic applicable to the diagnosed subject.

Thus, and as previously indicated, another aspect of the invention is a method of deciding or recommending to initiate a therapy (i.e., a medical regimen) for a subject suspected of suffering from a neurodevelopmental disorder, in which disorder one or more of neurogenesis, neural migration, and synaptogenesis events is impaired in relation to the absence of neurodevelopmental disorder, preferably suffering from Rett syndrome, which method comprises the steps of:
(a) carrying out the diagnostic method as defined in any one of the previous aspects or corresponding embodiments;
(b) optionally determining the outcome of a physical motor movement inspection and/or cardiac function and/or respiratory function and/or swallowing function; and
(c) if the subject is diagnosed of suffering from a neurodevelopmental disorder, and optionally of the stage of the disorder, then initiation of a treatment is recommended for the neurodevelopmental disorder.

In a particular embodiment, the method of deciding or recommending to initiate a therapy (i.e., a medical regimen) for a subject suspected of suffering from a neurodevelopmental disorder, is a method of deciding or recommending to initiate a therapy for Rett syndrome.

Particular treatments are then selected from one or more of gene therapy, symptom-based treatments selected from physical therapy, speech-language therapy, braces in case of scoliosis, beta-blockers in case of abnormal electrocardiogram, and rebreathing techniques in case of abnormal respiratory function.

This particular embodiment could be drafted as a method of treating a patient suffering from a neurodevelopmental disorder, in which disorder one or more of neurogenesis, neural migration, and synaptogenesis events is impaired in relation to the event in case of no neurodevelopmental disorder, said method comprising carrying out the in vitro method for the diagnosis and/ or prognosis as previously disclosed; and treating the patient with the appropriate medical regimen for the neurodevelopmental disorder.

In another particular embodiment, the method of treatment comprises:
(a) to apply a therapy appropriate for the neurodevelopmental disorder;
(b) to carry out the method for the analysis of the progression of a disorder, and/or for the monitoring of a treatment of a subject previously diagnosed of a neurodevelopmental disorder, wherein the expression level of one or more of a miRNA of the miR-302/367 cluster, and/or the expression level of one or more of a miRNA of the chromosome 14 miRNA cluster (C14MC) are determined at two or more time points;
(c) to determine the progression of the disorder and/or the effect of a monitored treatment by evaluating the relative variation of the expression level of the one or more of the determined miRNA of the miR-302/367 and/or C14MC clusters within the two or more time points; wherein (i) if this relative variation within the two or more time points is indicative of progression of disease and/or of non-effectivity of a monitored treatment, the patient is selected for a different therapy than in step (a), and (ii) if this relative variation within the two or more time points is indicative of no progression of disease and/or of effectivity of a monitored treatment, the patient is selected for a therapy selected from the continuing of therapy of step (a) and an additional or alternative therapy.

The invention also generically encompasses a method of detecting, in an isolated sample of a subject, the level of expression of at least one or more of a miRNA of the miR-302/367 cluster and/or at least one or more of a miRNA of the chromosome 14 miRNA cluster (C14MC). In particular the one or more miRNAs are determined in isolated EVs of the isolated sample. Also in particular, the said method of detecting comprises:
(a) isolating extracellular vesicles (EV) from an isolated sample of a subject, preferably from a biological fluid;
(b) collecting miRNA from the EVs, preferably in a buffered solution; and
(c) detecting if one or more of the miRNA is present, and/or which is its expression level, preferably by (i) contacting said collected miRNAs with means capable of binding the corresponding expressed miRNAs and detecting said binding.

The means capable of binding the corresponding expressed miRNAs are, in particular, probes (i.e., oligonucleotides) that hybridize with the miRNAs, preferably probes labeled with a compound detectable by spectroscopic means, in particular fluorescent and luminescent compounds. In another example, the means capable of binding the corresponding expressed miRNAs are, in particular, primers (i.e., oligonucleotides) that hybridize with the miRNAs and allow for a polymerized chain reaction (PCR).

In also another particular embodiment of the in vitro method of the invention for the diagnosis and/or prognosis of neurodevelopmental disorders as previously disclosed, after the determination of the expression level of the one or more of the miRNA or of the ratio of the said expression levels, the following computer-implemented steps are carried out: said level(s) or ratio(s) are given a value and/or a score, and optionally are computed in a mathematical formula to obtain a computed value; wherein in function of the said level(s), score(s) and or computed value(s), a decision is taken between the options of suffering or not from neurodevelopmental disorder, in particular of suffering or not from Rett syndrome, and/or between the progression of disorder, and/or efficacy of a treatment, and/or decision of recommending initiating a treatment.

In another particular embodiments of the in vitro methods of the invention that provide a diagnostic, a prognostic, an analysis of the progression of disorder, or of the effectivity of a therapy, or of the recommended therapy, they further comprise the steps of (i) collecting the information, and (ii) saving the information in a data carrier.

In the sense of the invention a "data carrier" is to be understood as any means that contain meaningful information data for the diagnostic, a prognostic, an analysis of the progression of disorder or of the effectivity of a therapy, such as paper. The carrier may also be any entity or device capable of carrying the information. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the diagnosis/therapy selection data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

For the carry out of the methods of the invention, commercial kits and conventional techniques widely known by the skilled person can be used. Moreover, the inventors do also provide for new kits developed with the aim of simplifying the execution of the methods.

Thus, in another aspect, the invention relates to the use of a kit comprising means for determining the expression level of one of the miRNA of the miR-302/367 cluster and/or of at least one of the miRNA of C14MC, and optionally means for collecting miRNA from EVs, for the diagnosis and/or prognosis of a neurodevelopmental disorder, in particular for the diagnosis and/or prognosis of Rett syndrome.

Is also herewith provided the use of a kit comprising means for determining the expression level of one of the miRNA of the miR-302/367 cluster and/or of at least one of the miRNA of C14MC, and optionally means for collecting miRNA from EVs, for the analysis of the progression of a neurodevelopmental disorder and/or for the monitoring of a treatment of a subject previously diagnosed of a neurodevelopmental disorder, in for the progression and/or monitoring of Rett syndrome.

And also another aspect is a kit comprising a means for determining the expression level of at least one of the miRNA of the miR-302/367 cluster and/or of at least one of the miRNAs of C14MC, and optionally means for collecting miRNA from EVs, wherein the means are at least 10% of the means in the kit for determining the expression level of the indicated mRNAs.

In a more particular embodiment of the kits of the invention, the means for determining the expression level of at least one of the miRNA of the miR-302/367 cluster and/or of at least one of the miRNAs of C14MC are at least 20%, at least 30%, at least 40%, at least 50%, at least 60%, at least 70%, at least 80%, at least 90% or at least 100% of the total amount of reagents for assaying miRNA forming the kit. Thus, in the particular case of kits comprising reagents (i.e., means) for assaying the levels of expression of at least one of the miRNA of the miR-302/367 cluster and/or of at least one of the miRNA of C14MC, the means specific for said biomarkers, that comprise in particular primers and probes, comprise at least 10%, at least 20%, at least 30%, at least 40%, at least 50%, at least 60%, at least 70%, at least 80%, at least 90% or at least 100% of the reagents, in particular primers and probes present in the kit. These kits are, thus, simplified kits including mainly the reagent means for detecting the levels of the one or more of the miRNAs.

In a particular embodiment, the probes comprised in the kits of the invention are labeled oligonucleotides that that hybridize with the miRNAs, preferably the probes comprise a compound detectable by spectroscopic means, in particular fluorescent and luminescent compounds. In another embodiment of the kits of the invention, the primers are also oligonucleotides that hybridize with the miRNAs and allow for a polymerized chain reaction (PCR).

Another aspect of the invention is the use of the expression level in extracellular vesicles (EVs) of at least one or more of a miRNA of the miR-302/367 cluster and/or of at least one or more of a miRNA of the chromosome 14 miRNA cluster, as marker of clinical diagnosis and/or prognosis of a neurodevelopmental disorder and/or for the analysis of the progression of the disorder and/or the monitoring of a treatment for the disorder, preferably for Rett syndrome.

Particular embodiments disclosed above for the in vitro methods do also apply to this aspect. In a particular embodiment of the use, it comprises the use of the expression level of a set of miRNA that comprises at least one or more of a miRNA of the miR-302/367 cluster and at least one or more of a miRNA of the chromosome 14 miRNA cluster (C14MC); and step (d) comprises comparing the said transcription levels to one or several reference transcription levels or range of reference transcription levels.

Herewith disclosed is an in vitro method for the diagnosis and/or prognosis of a neurodevelopmental disorder, in particular a brain neurodevelopmental disorder, in which one or more of neurogenesis, neural migration, and synaptogenesis events is impaired in relation to the event in case of no neurodevelopmental disorder, preferably for the diagnosis and/or prognosis of Rett syndrome, in which the expression level of one or more miRNA of the miR-302/367 cluster selected from the group consisting of hsa-miR-302a-3p, hsa-miR-302d-3p, hsa-miR-302c-5p and hsa-miR-302a-5p is determined in an isolated sample of a subject, being all the miRNA clusters defined according to the miRBase Release 22.1 of the miRBase.

In a particular embodiment of this aspect, the method comprises:
(a) isolating extracellular vesicles (EV) from an isolated sample of a subject;
(b) collecting miRNA from the EVs;
(c) determining the expression level of one or more miRNA of the miR-302/367 cluster selected from the group consisting of hsa-miR-302a-3p, hsa-miR-302d-3p, hsa-miR-302c-5p and hsa-miR-302a-5p; and
(d) comparing the said expression level with one or several reference expression levels,

wherein the subject is diagnosed of a neurodevelopmental disorder, preferably of Rett syndrome, when the expression levels of at least one of the miRNA of the miR-302/367 cluster is equal to a reference expression level of a subject suffering from the neurodevelopmental disorder, preferably of a subject suffering from Rett syndrome, for that miRNA of the respective clusters, or
wherein the subject is diagnosed of not suffering a neurodevelopmental disorder, preferably of Rett syndrome, when the expression level of the determined miRNA of the miR-302/367 cluster is not equal to the expression level of a subject suffering from the neurodevelopmental disorder, preferably of a subject suffering from Rett syndrome.

Herewith disclosed is an in vitro method for the diagnosis and/or prognosis of a neurodevelopmental disorder, in particular a brain neurodevelopmental disorder, in which one or more of neurogenesis, neural migration, and synaptogenesis events is impaired in relation to the event in case of no neurodevelopmental disorder, preferably for the diagnosis and/or prognosis of Rett syndrome, in which one more miRNA of C14MC selected from the group consisting of hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p is determined in an isolated sample of a subject, being all the miRNA clusters defined according to the miRBase Release 22.1 of the miRBase.

In a particular the method that comprises determining one more miRNA of C14MC, the method comprises:
(a) isolating extracellular vesicles (EV) from an isolated sample of a subject;
(b) collecting miRNA from the EVs;
(c) determining the expression level of the one or more of a miRNA of the chromosome 14 miRNA cluster (C14MC); and
(d) comparing the said expression level to one or several reference expression level(s),

wherein the subject is diagnosed of a neurodevelopmental disorder, preferably of Rett syndrome, when the expression level of at least one of the miRNA of C14MC is equal to a reference expression level of a subject suffering from the neurodevelopmental disorder, preferably of a subject suffering from Rett syndrome, for that miRNA of the respective clusters, or
wherein the subject is diagnosed of a neurodevelopmental disorder, preferably of Rett syndrome, when the expression level of at least one of the miRNA of C14MC is not equal to a reference expression level of a subject suffering from the neurodevelopmental disorder, preferably of a subject suffering from Rett syndrome.

An in vitro method for the diagnosis and/or prognosis of a neurodevelopmental disorder, in which one or more of neurogenesis, neural migration, and synaptogenesis events is impaired in relation to the event in case of no neurodevelopmental disorder, preferably for the diagnosis and/or prognosis of Rett syndrome, in which the expression level of one or more miRNA of the miR-302/367 cluster selected from the group consisting of hsa-miR-302a-3p, hsa-miR-302d-3p, hsa-miR-302c-5p and hsa-miR-302a-5p; and/or one or more miRNA of C14MC selected from the group consisting of hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p is determined in an isolated sample of a subject, being all the miRNA clusters defined according to the miRBase Release 22.1 of the miRBase.

In a particular the method comprises:
(a) isolating extracellular vesicles (EV) from an isolated [biological fluid] sample of a subject;
(b) collecting miRNA from the EVs;
(c) determining the expression level of the one or more miRNA of the miR-302/367 cluster and/or the one or more of a miRNA of the chromosome 14 miRNA cluster (C14MC); and
(d) comparing the said expression level(s) to one or several reference expression level(s),

wherein the subject is diagnosed of a neurodevelopmental disorder, preferably of Rett syndrome, when the expression level of at least one of the miRNA of the miR-302/367 cluster and/or when the expression level of at least one of the miRNA of C14MC is equal to a reference expression level of a subject suffering from the neurodevelopmental disorder, preferably of a subject suffering from Rett syndrome, for that miRNA of the respective clusters, or
wherein the subject is diagnosed of a neurodevelopmental disorder, preferably of Rett syndrome, when the expression level of the determined miRNA of the miR-302/367 cluster and/or when the expression level of the determined miRNA of the C14MC is not equal to a reference expression level of a subject suffering from the neurodevelopmental disorder, preferably of a subject suffering from Rett syndrome.

More in particular in any of the in vitro methods comprising the determining of the expression level of one or more of a miRNA from miR-302/367 cluster and/or C14MC, the expression level of one or more of hsa-miR-423-3p, hsa-miR-4488, hsa-miR-3960, hsa-miR-363-3p, hsa-miR-21-5p, hsa-miR-374b-5p, hsa-miR-19a-3p, hsa-miR-9-3p, and hsa-miR-335-3p is further determined.

Also, more in particular in any of the in vitro methods comprising the determining of the transcription profile of one or more of a miRNA from miR-302/367 cluster or C14MC, the isolated sample of the subject is a body fluid, in particular selected from the group consisting of blood, plasma, serum, urine, sputum, and cerebrospinal fluid.

### EXAMPLES

The following illustrative example is provided for the purpose of a better understanding of the invention, but it is not to be conceived as limiting.

Following the procedure illustrated in FIG. 2, region-specific forebrain organoids were generated from female hiPSCs with a MeCP2:R225X mutation and the corresponding isogenic control. EV miRNA and protein expression profiles in EVs were characterized at day 0, day 13, day 40, and day 75.

The data in FIG. 1 show that at least four members of the hsa-miR-302/367 cluster (i.e., hsa-miR-302a-3p, hsa-miR-302d-3p, hsa-miR-302c-5p and hsa-miR-302a-5p) exhibited an altered expression profile in the RTT forebrain organoids as compared to their isogenic controls at day 75. In particular, hsa-miR-302a-3p, hsa-miR-302d-3p showed a lower EV expression in the dorsal region; and hsa-miR-302c-5p and hsa-miR-302a-5p showed an upregulated EV expression in ventral region in Rett Syndrome brain organoids.

Moreover, a strong upregulation was found among the miRNA species of the chromosome 14 miRNA cluster (C14MC) in RTT forebrain organoids at all time points in both the ventral and dorsal regions.

Although data not shown, several members of the hsa-miR-302/367 cluster were identified as having a decreasing expression profile over time in both the ventral and dorsal regions during normal development (data derived from isogenic controls).

These results suggest, thus, essential roles of the hsa-miR-302/367 cluster and the C14MC in EVs during normal and RTT-associated neurodevelopment. Thus, EV miRNAs are useful biomarkers for diagnosing and monitoring RTT progression.

### Materials and Methods

### hiPSC lines and maintenance

A patient-derived hiPSC cell line, EMC24i/R2 (C6), with a nonsense mutation at R255X with C to T transition (RTT) and the respective isogenic (IC) cell line EMC24i/R2 (C5) were used as described by Gomes et al. (2020) (Gomes AR, Fernandes TG, Vaz SH, Silva TP, Bekman EP, Xapelli S, et al. Modeling Rett Syndrome With Human Patient-Specific Forebrain Organoids. Front Cell Dev Biol. 2020;8:610427. doi:10.3389/fcell.2020.610427). The nonsense mutation is located within the transcription repression domain - nuclear localization signal (TRD-NLS) region in the MeCP2 protein. Cells were cultured and maintained on MatrigelTM (Corning) - coated plates in serum-free mTeSRTM1 Plus medium and passaged every 2-3 days using 0.5 mM EDTA dissociation buffer (Thermofisher Scientific) when reached 85% confluency. iPSC clones were expanded two to three passages before starting the differentiation process.

### Induction of Region-Specific (Dorsal and Ventral) Forebrain Organoids

The protocol published by Gomes et al. (2020) and as summarized in Figure 2 was adopted. In brief, after 30 min of incubation at 37°C with ROCK inhibitor (ROCKi, Y-27632, 10 µM, StemCell Technologies), hiPSC colonies were dissociated using accutase (Sigma). Cells were then seeded (1.5 × 106 cells/well) on microwell plates (AggreWell TM 800, StemCell Technologies) in mTeSRTM1 Plus supplemented with 10 µM ROCKi for 24h. The expansion medium was then refreshed entirely without ROCKi supplement. Aggregates usually attain a diameter of 250-300 um within 2-3 days after which the medium is half changed to induction medium and this day is considered as day 0. The neural induction medium (N2B27) contained 50% of DMEM/F12/N2 (DMEM-F12, (Thermofisher Scientific) supplemented with 1% (v/v) N2 (Thermofisher Scientific), 1.6 g/L Glucose (Sigma), 1% (v/v) PenStrep, and 20 µg/mL Insulin (Sigma) and 50% of Neurobasal/B27 [Neurobasal medium (Thermofisher Scientific) supplemented with 2% (v/v) B27(-Vitamin A)-supplement (Thermofisher Scientific), 2 mM L-glutamine (Thermofisher Scientific) and 1% (v/v) PenStrep. For the dorsal patterned aggregates, the medium was further supplemented with 2 µM Dorsomorphine (Sigma) and 2 µM A83-01 (Tocris) until day 5 with half-medium being refreshed at days 0, 3, and 5. For the ventral forebrain patterning, 10 µM SB-431542 (SB) (Sigma) and 100 nM LDN-193189 (LDN) (Sigma) were added to the medium. At day 5, Cell aggregates (with a population of around 300 aggregates per well of the microwell plate) were transferred to Ultra-Low attachment 6-well plates (Corning). At day 7, the dorsal forebrain culture medium was half changed with fresh medium supplemented with 1 µM CHIR-99021 (tebu-bio), 10 µM SB-431542 (Sigma) and 10 µg/ml Heparin (Sigma) and the ventral forebrain culture medium was half changed with fresh medium supplemented with 2.5 µM IWP2 (Sigma), 100 nM SAG (Millipore) and 10 µg/ml Heparin (Sigma).

### Maintenance and Maturation of Dorsal and Ventral Forebrain Organoids

At day 13, the culture medium was half changed with N2B27 (+Vitamin A) without adding further dorsal and ventral supplemental reagents. This process was continued until day 40 with the medium being changed every 2-3 days. On day 41, the medium was changed to BrainPhysTM Neuronal Medium (StemCell Technologies), supplemented with NeuroCultTM SM1 Neuronal Supplement (StemCell Technologies), N2 Supplement-A (StemCell Technologies), Recombinant Human Brain Derived Neurotrophic Factor (BDNF, PeproTech, 20 ng/mL), Recombinant Human Glial-Derived Neurotrophic Factor (GDNF, PeproTech, 20 ng/mL), dibutyryl cAMP (1 mM, Sigma), and ascorbic acid (200 nM, Sigma). Organoids were maintained for 75 days with every 2-3 days changing one third of the total medium.

### EV Extraction and Characterization

### EVs purification

Serum-free conditioned medium was collected from iPSC/organoid cultures that were maintained in vitro for 2 days. Conditioned medium was first centrifuged at 400 ×g for 5 min to remove cells and cell debris. The supernatant was then centrifuged at 2000 ×g for 10 min to purify the medium from apoptotic bodies. The supernatant then underwent differential ultracentrifugation (Beckman, Germany) for 24 min at 10,000 ×g followed by filtration through a 0.22 µm filter (GVA, Millipore). The filtered supernatant was further centrifuged at 100,000 ×g for 47 min. The pellet was resuspended in 1 ml fresh PBS and transferred to MLA-150 tube. To collect the EVs, the final ultracentrifugation was performed using optimaTM MAX-XP tabletop ultracentrifuge (Beckman Coulter, USA) for 25 min at 100,000 ×g. All the centrifugation steps were carried out at 4°C. The pellet was then resuspended in elusion buffer provided within High Pure RNA Isolation Kit (Roche) and the total RNA was extracted and stored at -80°C until further procedure for transcriptomics.

### Nanoparticle tracking analysis (NTA)

To analyze EVs size distribution and concentration, NTA was performed using the ZetaView^{®} nanoparticle tracking analyzer (PMX120, Particle Metrix GmbH,Germany ) equipped with software version 8.05.11_SP4. First, the instrument was calibrated with 100nm polystyrene standard beads with the suggested concentration according to manufacturer's instruction. EV samples were diluted to 1 ml in PBS prior to analysis and measured at 11 positions/cycle.

### MiRNA Expression Profiling

The generation of miRNA expression data from the extracted total RNA was performed by LC Sciences (Houston, TX, USA) and consisted of the analysis of RNA quality and quantity (Bioananalyzer 2100, Agilent, CA, USA), the preparation of small RNA library (TruSeq Small RNA Sample Prep Kits, Illumina, San Diego, USA), and single-end sequencing (Illumina Hiseq 2500).

Furthermore, the initial data pre-processing included the deletion of low-quality reads, 3' adapter sequences, and contaminations as well as the removal of other non-coding RNA (rRNA, tRNA, snRNA, and snoRNA) and degradation fragments of mRNA. The remaining sequences were mapped to human miRNA precursors in miRBase 22.1, allowing for length variation at the 3' and 5' ends and one mismatch in the aligned sequence (LC Sciences, Houston, TX, USA).

Subsequently, the filtering of lowly expressed miRNAs and trimmed mean of M values (TMM) and counts per million (CPM) normalization was performed in-house in accordance with a established edgeR workflow. Boxplots, density plots, and principal component analysis (PCA) plots were constructed for the evaluation of the pre-processed data quality.

### Characterization of Temporal Dynamics in miRNA Expression

The quasi-likelihood F-test from the edgeR package (Robinson MD, McCarthy DJ, Smyth GK. edgeR: a Bioconductor package for differential expression analysis of digital gene expression data. Bioinformatics. 2010;26(1):139-40. doi:10.1093/bioinformatics/btp616) was used to identify miRNAs that are statistically significantly changed (i.e., false discovery rate (FDR)-adjusted p-value < 0.05) between at least two time points in the IC samples. This was done separately for the samples from the ventral and dorsal regions. These significant miRNAs were subsequently clustered based on their unit-scaled log2 CPM value using the Euclidean distance and Ward's minimum variance linkage method. Besides identifying miRNAs with a dynamic expression profile over time, miRNAs with a ubiquitous expression - defined as a normalized log2 CPM value of at least 12 in all IC samples - were identified as well.

### Identification of miRNA Expression Alterations in Rett Syndrome

To identify miRNAs with a differential expression between RTT and IC samples, the quasi-likelihood F-test from the edgeR package (Robinson 2010) was applied at each time point for the two groups, separately for the samples from the dorsal and ventral regions. The miRNAs with an FDR-adjusted p-value < 0.05 were considered to be significantly differentially expressed at that time point.

### Pathway Analysis

The miRNA-target interaction data from miRTarBase v9.0 was used to get the target genes of all measured miRNAs. Accordingly, using the curated pathway gene sets from WikiPathways (August 10, 2022) (36), overrepresentation analysis (clusterProfiler package (37)) was separately performed on the union of the target genes of ubiquitously expressed miRNAs, the miRNAs with a significantly changed expression between at least two time points, and the miRNAs with a differential expression between RTT and IC samples at any time point. In addition, to reliably estimate the pathways' significances, 10,000 random miRNA sets with the same set size as the total number of significant miRNAs were used to empirically estimate the distribution of each pathway's significance under the null hypothesis and to calculate the permutation p-value.

### Results

### Characterization of EVs and Sample Quality

The NTA results demonstrated the presence of EVs with a mean diameter of 130 nm for both RTT (MeCP2:R255X) and IC, which is within the range reported for EVs. Furthermore, the protein expression data was used to evaluate the presence of known exosomal makers in the extracted EVs. The top-ranked exosomal protein markers from ExoCarta showed high expression of EV protein markers in our samples. These include ESCRT proteins such as PDCD6IP, also known as ALIX, HSP90, HSP70 (HSPA8), and tetraspanins including CD9, CD81 as well as RAB proteins. Good quality was reported.

### Temporal Dynamics of Extracellular Vesicle miRNA Expression

To evaluate the changes in miRNA expression during normal brain development, statistical analysis was performed to identify the miRNAs with an altered expression between at least two time points in the IC samples. From this statistical analysis, twenty miRNAs were found to be differentially expressed. The hierarchical clustering of these miRNAs resulted in three miRNA clusters with distinct temporal expression profiles.

WikiPathway overrepresentation analysis of the clusters - which encompass miRNAs with a, respectively, decreasing and increasing expression overtime - did not yield any significant pathways after FDR adjustment. However, the Notch Signaling Pathway (WP61) was the most significantly enriched pathway of the decreasing cluster (permutation p-value = 5e-4), while three JAK/STAT-signaling-related pathways (i.e., EGFR tyrosine kinase inhibitor resistance (WP4806), Prolactin signaling pathway (WP2037), Leptin-insulin signaling overlap (WP3935)) formed the top most significantly enriched pathways of the increasing cluster (permutation p-value = 3e-4, for all three pathways).

Furthermore, ten ubiquitously expressed miRNAs with a log2 CPM of at least 12 among all IC samples were identified as well. The WikiPathways overrepresentation analysis on this gene set yielded no significant results after FDR adjustment.

### Decreasing Expression over Time of the hsa-miR-302/367 Cluster

From the twenty miRNAs with a significantly different expression over time in the IC samples, six species belonged to the hsa-miR-302/367 cluster located on chromosome 4 (4q25 region). Noteworthily, all members of this cluster have a highly correlated expression profile, exhibiting a decreasing expression over time in both dorsal and ventral regions of the IC samples (data not shown). Furthermore, when comparing the expression of the IC and RTT forebrain organoids, four members of the hsa-miR-302/367 cluster were found to be significantly up- or downregulated at day 75 in the dorsal or ventral region of RTT forebrain organoids, in particular the miRNAs hsa-miR-302a-3p, hsa-miR-302d-3p, hsa-miR-302c-5p and hsa-miR-302a-5p (FIG. 1, see miRNAs in black borders).

### Elevated Expression of the Chromosome 14 miRNA Cluster in RTT

Twenty-nine miRNAs were differentially expressed between RTT and IC samples in at least one time point. WikiPathways overrepresentation analysis (data not shown). On the set of differentially expressed miRNAs, the Serotonin HTR1 group and FOS pathway (WP722) was the most significantly enriched pathway with an (unadjusted) permutation p-value of 1.6e-3. Interestingly, more than half of the differentially expressed miRNAs belong to the chromosome 14 miRNA cluster (C14MC) located at the chr14q32 region. Although not all members of this cluster reached statistical significance, an overall higher expression in the RTT samples across the entire cluster was observed. In particular an overall higher expression in the RTT samples was observed for hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p in any of the dorsal or ventral regions at least one of the assayed time points (FIG. 1, see miRNAs in black borders and also in the box at the left of the figure).

### Discussion

In the present study, the time-dependent EV expression profiles in region-specific MeCP2:R255X (RTT) and IC forebrain organoids generated from patient-derived iPSCs was characterized. Hereby, several miRNA species and clusters were identified that potentially play a key role in normal and Rett Syndrome-affected neural development.

### EV miRNAs with a Dynamic Temporal Expression Profile

### MiRNAs with increasing EV expression during neural development

Several miRNAs with an increasing EV expression profile during the progression of neural development were found, which have before been identified as key players in neural differentiation. For instance, hsa-miR-125 and hsa-miR-9 have previously been suggested to promote the differentiation of iPSCs to neural stem cells (Kulcenty K, Wroblewska JP, Rucinski M, Kozlowska E, Jopek K, Suchorska WM. MicroRNA Profiling During Neural Differentiation of Induced Pluripotent Stem Cells. Int J Mol Sci. 2019;20(15). doi:10.3390/ijms20153651). In addition, hsa-miR-99a, another miRNA with an increasing EV expression (data not shown), has also been shown to be upregulated during in vitro neural differentiation (Stevanato L, Sinden JD. The effects of microRNAs on human neural stem cell differentiation in two- and three-dimensional cultures. Stem Cell Res Ther. 2014;5(2):49. doi:10.1186/scrt437). Furthermore, Peng et al. (2020) and Li et al. (2019) demonstrated the anti-proliferative potential of hsa-miR-30a and hsa-miR-7-5p (data not shown), respectively, in glioma (stem) cells (Peng L, Ming Y, Zhang L, Zhou J, Xiang W, Zeng S, et al. MicroRNA-30a suppresses self-renewal and tumorigenicity of glioma stem cells by blocking the NT5E-dependent Akt signaling pathway. FASEB J. 2020;34(4):5128-43. doi:10.1096/fj.201802629RR; and Li G, Huang M, Cai Y, Yang Y, Sun X, Ke Y. Circ-U2AF1 promotes human glioma via derepressing neuro-oncological ventral antigen 2 by sponging hsa-miR-7-5p. J Cell Physiol. 2019;234(6):9144-55. doi:10.1002/jcp.27591). The top three pathways enriched with the target genes of these miRNAs with an increasing expression over time, all include JAK/STAT signaling as part of their diagrams, a signaling pathway that has previously been shown to regulate the expression of genes involved in neurogenesis as well as synaptic plasticity and transmission in primary cultured neurons (Hixson KM, Cogswell M, Brooks-Kayal AR, Russek SJ. Evidence for a non-canonical JAK/STAT signaling pathway in the synthesis of the brain's major ion channels and neurotransmitter receptors. BMC Genomics. 2019;20(1):677. doi: 1 0.1186/s 12864-0 19-6033-2). Together, the miRNAs with an observed increasing EV expression over time may be anti-proliferative and promote the differentiation of recipient cells into mature neurons.

### Decreasing EV expression of the hsa-miR-302/367 cluster during neural development

Additionally, in the IC samples, members of the hsa-miR-302/367 cluster were found to have a decreasing EV expression during the progression of neural development (data not shown).

This miRNA cluster is located on human chromosome 4 within the intron of the long non-coding gene MIR302CHG and the coding gene LARP7 in the sense and antisense direction, respectively. Genetic variants in these genes have been associated with Alazami Syndrome, a disorder characterized by growth restriction and intellectual disability, suggesting an important role of this genomic region in (neural) development. Indeed, the hsa-miR-302/367 cluster has previously been shown to control ectodermal differentiation (Sugawara T, Miura T, Kawasaki T, Umezawa A, Akutsu H. The hsa-miR-302 cluster controls ectodermal differentiation of human pluripotent stem cell via repression of DAZAP2. Regen Ther. 2020;15:1-9. doi:10.1016/j.reth.2020.03.011) and to be necessary for the maintenance of pluripotency of iPSCs. Furthermore, Kulcenty et al. (2019, *supra)* showed that, compared to iPSCs, hsa-miR-302 is downregulated in neural stem cells. Besides the hsa-miR-302/367 cluster, hsa-miR-92a, which is another known promoter of cellular proliferation, also showed a decreasing expression over time. In the present study, the high EV expression of these miRNAs in the early stages of neural development might thus promote proliferation and the maintenance of pluripotency of neighboring cells. Interestingly, the Notch signaling pathway was found to be the most significantly enriched pathway by miRNAs with a decreasing EV expression over time, indicating that the modulation of this pathway by EV miRNAs is predominantly active during the early stages of neural development. Indeed, by regulating both neural differentiation and proliferation, the Notch signaling pathway is known to be a key regulatory pathway during early neurogenesis (Zhou B, Lin W, Long Y, Yang Y, Zhang H, Wu K, et al. Notch signaling pathway: architecture, disease, and therapeutics. Signal Transduct Target Ther. 2022;7(1):95. doi:10.1038/s41392-022-00934-y), emphasizing the need for an active miRNA-dependent regulation during these early stages.

### Expression changes of the hsa-miR-302/367 cluster in RTT

Besides a decreasing expression over time during normal neural development, a significantly lower EV expression of two members of the hsa-miR-302/367 cluster after 75 days in the dorsal region of the RTT brain organoids was found as compared to their isogenic controls (hsa-miR-302a-3p, and hsa-miR-302d-3p). As the hsa-miR-302/367 cluster is required for the maintenance of pluripotency, the downregulation of these miRNAs may lead to the early differentiation of neural cells in the dorsal region. In line with our findings, through immunocytochemistry and flow cytometry analyses, Gomes et al. (2020) *(supra)* also identified premature differentiation in the dorsal region of Rett Syndrome brain organoids. In contrast, after 75 days in the ventral region, two members of the hsa-miR-302/367 cluster (hsa-miR-302c-5p and hsa-miR-302a-5p) were found with a significantly upregulated EV expression in Rett Syndrome brain organoids. Due to the important role of the hsa-miR-302/367 in the preservation of cellular pluripotency, the upregulation of this miRNA cluster may inhibit late neural differentiation in the ventral region. As cellular differentiation is often needed for neural migration, the upregulated EV expression of the hsa-miR-302/367 miRNA cluster might be partially responsible for the impaired neural migration in the ventral region of Rett syndrome brain organoids as observed by Gomes et al. (2020).

### EV Expression of the Chromosome 14 miRNA Cluster

### Elevated expression of C14MC in RTT

Besides the miR-302/367 cluster, the results also suggest an important role of the Chromosome 14 miRNA Cluster (C14MC) in Rett Syndrome pathology. Specifically, an overall higher expression in RTT versus IC of most members of this cluster in RTT brain organoids was observed, with 15 of the 50 miRNAs reaching statistical significance (FIG. 1).

C14MC is one of the largest clusters of miRNAs in the human genome located at the imprinted DLK1-DIO3 domain on the long arm of chromosome 14 (14q32). C14MC has been considered as a pregnancy-related cluster with a crucial role in placental development.

These cluster miRNAs are implicated in the regulation of various cellular processes, including proliferation, differentiation, and apoptosis. Their dysregulations have also been reported in various cancer types as well as neurological disorders.

Furthermore, the miRNAs within this cluster regulate a set of genes critical for growth and brain development. For instance, miR-329-3p was shown to regulate neural stem cell proliferation by inhibiting E2F1 expression (Lin D, Shi Y, Hu Y, Du X, Tu G. miR-329-3p regulates neural stem cell proliferation by targeting E2F1. Mol Med Rep. 2019;19(5):4137-46. doi:10.3892/mmr.2019.10096). Another member of the cluster, miR-409-3p, was shown to have a functional role in refining neuronal cell fate within neocortical layer 5 by controlling intermediate neuronal progenitor (IPC) cell proliferation via targeting Cited2, a protein that is required for IPCs expansion in the subventricular zone (SVZ) (Wagner NR, Sinha A, Siththanandan V, Kowalchuk AM, MacDonald JL, Tharin S. miR-409-3p represses Cited2 to refine neocortical layer V projection neuron identity. Front Neurosci. 2022;16:931333. doi:10.3389/fnins.2022.931333). MiR-409-3p was further shown to support the corticospinal projection identity of neurons (Diaz JL, Siththanandan VB, Lu V, Gonzalez-Nava N, Pasquina L, MacDonald JL, et al. An evolutionarily acquired microRNA shapes development of mammalian cortical projections. Proc Natl Acad Sci U S A. 2020;117(46):29113-22. doi:10.1073/pnas.2006700117). The regulatory effect of miRNAs in C14MC on neurogenesis can be further expanded to other members. For instance, Rago et al. (2014) revealed the presence of the miR-379/miR-410 cluster, the murine homolog of human C14MC, in embryonic mouse forebrain at different developmental stages (Rago L, Beattie R, Taylor V, Winter J. miR379-410 cluster miRNAs regulate neurogenesis and neuronal migration by fine-tuning N-cadherin. EMBO J. 2014;33(8):906-20. doi:10.1002/embj.201386591). The authors demonstrated that miR-369-3p, miR-496, and miR-543 regulate proliferation and differentiation in neural progenitor cells and newborn migrating neurons in the developing neocortex by reducing N-cadherin expression levels.

Moreover, the function of the miR-379/410 cluster (C14MC) is essential for activity-dependent dendritic outgrowth in hippocampal neurons.

Another neurogenesis-related miRNA of the cluster is miR-381-3p. Our results indicate that mir-381-3p is consistently upregulated through all time points in MeCP2:R255X dorsal and ventral forebrain for days 0, 13, and 40 as compared to IC. Interestingly, Gomes et al. (2020) *(supra)* reported the premature transition of iPSCs into newborn neurons in MeCP2:R255X dorsal forebrain. This might suggest a potential role for the C14MC cluster in such alterations.

Regulation of synaptic transmission is another area where C14MC is actively involved. For example, the miR-379/410 cluster (C14MC) has been shown to regulate the expression of genes involved in synaptic activity and neural function (Whipple AJ, Breton-Provencher V, Jacobs HN, Chitta UK, Sur M, Sharp PA. Imprinted Maternally Expressed microRNAs Antagonize Paternally Driven Gene Programs in Neurons. Mol Cell. 2020;78(1):85-95 e8. doi:10.1016/j.molcel.2020.01.020). Synaptic-related events are also reported to be mediated by other members of the C14MC; miR-329-3p and miR-495-3p. These miRNAs regulate homeostatic synaptic depression (HSD) in excitatory neurons through inhibition of Prr7 mRNA translation to ensure the protection of excitatory neurons from over-excitation.

The activities of miRNAs within C14MC are not limited to neurodevelopment. They also ensure neuroprotection and confer anti-inflammatory effects, particularly after injuries such as cerebral ischemia and neuron or spinal cord injuries. For instance, in our study miR-410-3p overexpression was present in MeCP2:R255X dorsal region in all time points as compared to IC. Overexpression of miR-410-3p has a preventive effect against apoptosis and promotes neuronal survival in a hypoxic-ischemic brain damage (HIBD) model. Interestingly, Han et al. (2021) demonstrated that miR-410-3p from mesenchymal stem cell-derived EVs was able to delay neuronal apoptosis in a HIBD mouse model by increasing the expression of EGR2/Bcl2 via HDAC1(Han J, Yang S, Hao X, Zhang B, Zhang H, Xin C, et al. Extracellular Vesicle-Derived microRNA-410 From Mesenchymal Stem Cells Protects Against Neonatal Hypoxia-Ischemia Brain Damage Through an HDAC1-Dependent EGR2/Bcl2 Axis. Front Cell Dev Biol. 2020;8:579236. doi:10.3389/fcell.2020.579236).

Another overexpressed miRNA in MeCP2:R255X forebrain organoid was miR-379-5p, which has also a neuroprotective role by reducing autophagy of neurons in ischemic stroke via suppression of MAP3K2 and the JNK/c-Jun axis (Mo Y, Sun YY, Yue E, Liu Y, Liu KY. MicroRNA-379-5p targets MAP3K2 to reduce autophagy and alleviate neuronal injury following cerebral ischemia via the JNK/c-Jun signaling pathway. Kaohsiung J Med Sci. 2022;38(3):230-43. doi:10.1002/kjm2.12488). In addition to its role in neurogenesis described above, miR-381-3p overexpression also offers anti-inflammatory and anti-apoptosis effects via targeting CCR2 in ischemic brain injury. Other members of C14MC with a neuroprotective role after injuries include miR-494-3p, miR-136-3p, and miR-485-5p.

### Known involvement of C14MC in pathophysiology

Apart from its role during brain development, C14MC is also involved in the pathology of several neurological diseases including Alzheimer's disease (AD), Parkinson's disease (PD), amyotrophic lateral sclerosis (ALS), neuroblastoma, and epilepsy. Among the individual miRNAs within the cluster, miR-485 is the most well-studied with implications in both AD and PD as described in more detail by Ryu et al. (2023) (Ryu IS, Kim DH, Cho HJ, Ryu JH. The role of microRNA-485 in neurodegenerative diseases. Rev Neurosci. 2023;34(1):49-62. doi:10.1515/revneuro-2022-0039). Interestingly, the overexpression of miR-485-3p and miR-485-5p have also been observed in the exosomes isolated from cerebrospinal fluid (CSF) of patients with AD and PD. Furthermore, studies have reported involvement of miR-485 in peripheral nerve regeneration, protection against apoptosis by targeting the Rac1/Notch2 signaling pathway, regulation of neurite outgrowth, axonal development, and synapse formation, reduction of seizure frequency and the number of epileptiform spikes in drug-resistant epilepsy rat models, and apoptosis of glioma cells. Both mature miRNAs produced from miR-485 are significantly upregulated in our results pointing to the possible existence of common disrupted pathway(s) between RTT and other neurological diseases.

MiR-494-3p is another miRNA of the cluster with implications in PD. Geng et al. (2018) found that upregulation of miR-494-3p exacerbates motor impairment in a PD mouse model via targeting SIRT3 (Geng L, Zhang T, Liu W, Chen Y. miR-494-3p modulates the progression of in vitro and in vivo Parkinson's disease models by targeting SIRT3. Neurosci Lett. 2018;675:23-30. doi:10.1016/j.neulet.2018.03.037). There are also miRNAs of the cluster detected in ALS; Capauto et al. (2018) discovered a cross-talk between Gria2, miR-409, and miR-495 in ECS-derived motor neurons with a mutation in the FUS gene. They verified a circuitry in which miR-409 and miR-495 downregulate Gria2, a subunit of the glutamate α-amino-3-hydroxy-5-methyl-4-isoxazole propionic acid (AMPA) receptor, which has an essential role in excitatory neurotransmission (Capauto D, Colantoni A, Lu L, Santini T, Peruzzi G, Biscarini S, et al. A Regulatory Circuitry Between Gria2, miR-409, and miR-495 Is Affected by ALS FUS Mutation in ESC-Derived Motor Neurons. Mol Neurobiol. 2018;55(10):7635-51. doi:10.1007/s12035-018-0884-4).

One of the mechanisms by which a MECP2 loss-of-function mutation can potentially lead to the overexpression of the C14MC was explored by Wu et al. (2010), who identified the upregulation of 22 miRNAs species from the mouse homolog of the C14MC (i.e., Dlk2-Gtl2 imprinting domain) in Mecp2-KO mice (Wu H, Tao J, Chen PJ, Shahab A, Ge W, Hart RP, et al. Genome-wide analysis reveals methyl-CpG-binding protein 2-dependent regulation of microRNAs in a mouse model of Rett syndrome. Proc Natl Acad Sci USA. 2010;107(42):18161-6. doi:10.1073/pnas.1005595107). Additional analyses by the authors revealed several Mecp2 binding sites within this genomic region and an upregulation of histone H3/H4 acetylation in Mecp2-KO mice, suggesting that MECP2 can directly regulate the expression of miRNAs from the C14MC. In line with our results, their findings indicate that MECP2 loss-of-function enhances C14MC expression. A possible indirect mechanism by which MECP2 can regulate C14MC expression might relate to its reported negative regulation of the Mef2c gene in mice, a gene that was shown to promote the expression of multiple miRNAs from the mouse homolog of the C14MC. Hence, a loss-of-function mutation in the MECP2 gene might lead to the upregulation of MEF2C expression which in turn may promote C14MC transcriptional activity.

By reviewing the literature on the role of miRNAs in the brain, it is evident that C14MC is actively involved in two main areas: neurodevelopment, mainly during neurogenesis (this covers both regulation of proliferation and differentiation), as well as synaptic function. In line with these functions, C14MC exerts its impact on neurological conditions including mainly AD and PD, but more noticeably during brain damage, including hypoxia, cerebral/brain ischemia and apoptosis.

From this Examples derives that the use of region-specific brain organoids, which - by mimicking an in vivo human forebrain - is a more realistic model of the human brain compared to (fibroblast or neuronal) cell cultures as well as a good alternative to animal models. The use of brain organoids, therefore, allowed the inventors to gain novel insights into the molecular pathophysiology of RTT. Novel insights also came from the measurements at multiple developmental stages which enabled to investigate the temporal dynamics of EV miRNA expression in normal and RTT-associated brain development, something that to the best of the inventor's knowledge has never been done before. Finally, the use of isogenic controls - which share the same genetic background as the MECP2 mutant brain organoids - allowed to confidently attribute the found alterations in the EV miRNA expression profile to the mutated MECP2 gene.

### Citation list

- Sidhaye et al. 2021. Brain organoids: an ensemble of bioassays to investigate human neurodevelopment and disease. Cell Death & Differentiation. 28:52-67 https://doi.org/10.1038/s41418-020-0566-4
- Gomes AR, Fernandes TG, Vaz SH, Silva TP, Bekman EP, Xapelli S, et al. Modeling Rett Syndrome With Human Patient-Specific Forebrain Organoids. Front Cell Dev Biol. 2020;8:610427. doi:10.3389/fcell.2020.610427
- Robinson MD, McCarthy DJ, Smyth GK. edgeR: a Bioconductor package for differential expression analysis of digital gene expression data. Bioinformatics. 2010;26(1):139-40. doi:10.1093/bioinformatics/btp616.
- Kulcenty K, Wroblewska JP, Rucinski M, Kozlowska E, Jopek K, Suchorska WM. MicroRNA Profiling During Neural Differentiation of Induced Pluripotent Stem Cells. Int J Mol Sci. 2019;20(15). doi:10.3390/ijms20153651.
- Stevanato L, Sinden JD. The effects of microRNAs on human neural stem cell differentiation in two- and three-dimensional cultures. Stem Cell Res Ther. 2014;5(2):49. doi: 1 0.1186/scrt437
- Peng L, Ming Y, Zhang L, Zhou J, Xiang W, Zeng S, et al. MicroRNA-30a suppresses self-renewal and tumorigenicity of glioma stem cells by blocking the NT5E-dependent Akt signaling pathway. FASEB J. 2020;34(4):5128-43. doi:10.1096/fj.201802629RR.
- Li G, Huang M, Cai Y, Yang Y, Sun X, Ke Y. Circ-U2AF1 promotes human glioma via derepressing neuro-oncological ventral antigen 2 by sponging hsa-miR-7-5p. J Cell Physiol. 2019;234(6):9144-55. doi:10.1002/jcp.27591.
- Hixson KM, Cogswell M, Brooks-Kayal AR, Russek SJ. Evidence for a non-canonical JAK/STAT signaling pathway in the synthesis of the brain's major ion channels and neurotransmitter receptors. BMC Genomics. 2019;20(1):677. doi:10.1186/s12864-019-6033-2
- Zhou B, Lin W, Long Y, Yang Y, Zhang H, Wu K, et al. Notch signaling pathway: architecture, disease, and therapeutics. Signal Transduct Target Ther. 2022;7(1):95. doi:10.1038/s41392-022-00934-y
- Lin D, Shi Y, Hu Y, Du X, Tu G. miR-329-3p regulates neural stem cell proliferation by targeting E2F1. Mol Med Rep. 2019;19(5):4137-46. doi:10.3892/mmr.2019.10096
- Wagner NR, Sinha A, Siththanandan V, Kowalchuk AM, MacDonald JL, Tharin S. miR-409-3p represses Cited2 to refine neocortical layer V projection neuron identity. Front Neurosci. 2022;16:931333. doi:10.3389/fnins.2022.931333
- Diaz JL, Siththanandan VB, Lu V, Gonzalez-Nava N, Pasquina L, MacDonald JL, et al. An evolutionarily acquired microRNA shapes development of mammalian cortical projections. Proc Natl Acad Sci USA. 2020;117(46):29113-22. doi:10.1073/pnas.2006700117
- Rago L, Beattie R, Taylor V, Winter J. miR379-410 cluster miRNAs regulate neurogenesis and neuronal migration by fine-tuning N-cadherin. EMBO J. 2014;33(8):906-20. doi:10.1002/embj.201386591
- Whipple AJ, Breton-Provencher V, Jacobs HN, Chitta UK, Sur M, Sharp PA. Imprinted Maternally Expressed microRNAs Antagonize Paternally Driven Gene Programs in Neurons. Mol Cell. 2020;78(1):85-95 e8. doi:10.1016/j.molcel.2020.01.020
- Han J, Yang S, Hao X, Zhang B, Zhang H, Xin C, et al. Extracellular Vesicle-Derived microRNA-410 From Mesenchymal Stem Cells Protects Against Neonatal Hypoxia-Ischemia Brain Damage Through an HDAC1-Dependent EGR2/Bcl2 Axis. Front Cell Dev Biol. 2020;8:579236. doi:10.3389/fcell.2020.579236
- Mo Y, Sun YY, Yue E, Liu Y, Liu KY. MicroRNA-379-5p targets MAP3K2 to reduce autophagy and alleviate neuronal injury following cerebral ischemia via the JNK/c-Jun signaling pathway. Kaohsiung J Med Sci. 2022;38(3):230-43. doi: 1 0.1 002/kjm2.12488
- Ryu IS, Kim DH, Cho HJ, Ryu JH. The role of microRNA-485 in neurodegenerative diseases. Rev Neurosci. 2023;34(1):49-62. doi:10.1515/revneuro-2022-0039
- Geng L, Zhang T, Liu W, Chen Y. miR-494-3p modulates the progression of in vitro and in vivo Parkinson's disease models by targeting SIRT3. Neurosci Lett. 2018;675:23-30. doi:10.1016/j.neulet.2018.03.037
- Wu H, Tao J, Chen PJ, Shahab A, Ge W, Hart RP, et al. Genome-wide analysis reveals methyl-CpG-binding protein 2-dependent regulation of microRNAs in a mouse model of Rett syndrome. Proc Natl Acad Sci USA. 2010;107(42): 18161-6. doi:10.1073/pnas. 1005595107
- Capauto D, Colantoni A, Lu L, Santini T, Peruzzi G, Biscarini S, et al. A Regulatory Circuitry Between Gria2, miR-409, and miR-495 Is Affected by ALS FUS Mutation in ESC-Derived Motor Neurons. Mol Neurobiol. 2018;55(10):7635-51. doi:10.1007/s12035-018-0884-4

## Claims

1. An in vitro method for the diagnosis and/or prognosis of a neurodevelopmental disorder, in which disorder one or more of neurogenesis, neural migration, and synaptogenesis events is impaired in relation to the event in case of no neurodevelopmental disorder, preferably the method for the diagnosis and/or prognosis of Rett syndrome, wherein the method comprises:
(a) isolating extracellular vesicles (EV) from an isolated sample of a subject;
(b) collecting miRNA from the EVs;
(c) determining the expression level of at least one or more of a miRNA of the miR-302/367 cluster and/or at least one or more of a miRNA of the chromosome 14 miRNA cluster (C14MC); and
(d) comparing the said expression levels to one or several reference expression levels or range of reference expression levels,
wherein the subject is diagnosed of a neurodevelopmental disorder, when the expression of at least one of the miRNA of the miR-302/367 cluster and/or of at least one of the miRNA of C14MC are equal to a reference expression level of a subject suffering from the neurodevelopmental disorder for that miRNA of the respective clusters, or
wherein the subject is diagnosed of not suffering a neurodevelopmental disorder, when the expression level of the determined miRNA of the miR-302/367 and/or C14MC clusters are not equal to the expression level of a subject suffering from the neurodevelopmental disorder,
being all the miRNA in the clusters defined according to the miRBase Release 22.1 of the miRBase.

2. The in vitro method according to claim 1, wherein the diagnosis and/or prognosis is for the analysis of progression of disorder and/or for the monitoring of a treatment of a subject previously diagnosed of a neurodevelopmental disorder, preferably diagnosed of Rett syndrome, and wherein preferably the expression level of one or more of a miRNA of the miR-302/367 cluster and/or the level of one or more of a miRNA of the chromosome 14 miRNA cluster (C14MC) are determined at two or more time points, and the progression and/or the effect of a monitored treatment is determined by evaluating the relative variation of the expression level of the one or more of the miRNA of the miR-302/367 and/or C14MC clusters within the two or more time points.

3. The vitro method according to any one of claims 1-2, wherein step (c) comprises determining the expression level of a set of miRNA that comprises at least one or more of a miRNA of the miR-302/367 cluster and at least one or more of a miRNA of the chromosome 14 miRNA cluster (C14MC); and step (d) comprises comparing the said transcription levels to one or several reference transcription levels or range of reference transcription levels,

4. The in vitro method according to any one of claims 1-3, in which the one or more miRNA of the miR-302/367 cluster is selected from the group consisting of hsa-miR-302a-3p, hsa-miR-302d-3p, hsa-miR-302c-5p and hsa-miR-302a-5p.

5. The in vitro method according to any one of claims 1-4, in which the one or more miRNA of C14MC is selected from the group consisting of hsa-miR-329-3p, hsa-miR-543, hsa-miR-409-3p, hsa-miR-539-3p, hsa-miR-127-3p, hsa-miR-495-3p, hsa-miR-410-3p, hsa-miR-381-3p, hsa-miR-494-3p, hsa-miR-370-3p, hsa-miR-136-3p, hsa-miR-379-5p, hsa-miR-323a-3p, hsa-miR-485-5p, hsa-miR-485-3p, and hsa-miR-369-3p.

6. The in vitro method according to any one of claims 1-5, in which the expression level of further one or more of hsa-miR-423-3p, hsa-miR-4488, hsa-miR-3960, hsa-miR-363-3p, hsa-miR-21-5p, hsa-miR-374b-5p, hsa-miR-19a-3p, hsa-miR-9-3p, and hsa-miR-335-3p is determined.

7. The in vitro method according to any one of claims 1-6, in which the following expression profile is determined:
- in the miR-302/367 cluster, if one or more of hsa-miR-302a-3p and hsa-miR-302d-3p is down-expressed, and/or if one or more of hsa-miR-302c-5p and hsa-miR-302a-5p is up-expressed in relation to a subject not suffering a neurodevelopmental disorder; and
- in the C14MC, if one or more of the miRNA is up-expressed in relation to a subject not suffering neurodevelopmental disorder.

8. The in vitro method according to any one of claims 3-7, in which a ratio is established between the expression level of the one or more miRNA determined within the C14MC and the expression level of the one or more miRNA determined within the miR-302/367 cluster.

9. The in vitro method according to any one of claims 1-8, wherein the neurodevelopmental disorder is a neurogenic disorder selected from Rett syndrome, Fragile X syndrome, Down syndrome, and hypogonadotropic hypogonadal syndromes, preferably is Rett syndrome.

10. The in vitro method according to any one of claims 1-9, wherein the isolated sample of the subject is a body fluid, preferably selected from the group consisting of blood, plasma, serum, urine, sputum, and cerebrospinal fluid.

11. A method of deciding or recommending to initiate a therapy for a subject suspected of suffering from a neurodevelopmental disorder, in which disorder one or more of neurogenesis, neural migration, and synaptogenesis events is impaired in relation to the event in case of no neurodevelopmental disorder, preferably suffering from Rett syndrome, which method comprises the steps of:
(a) carrying out the diagnostic method as defined in any one of claims 1-10;
(b) optionally determining the outcome of a physical motor movement inspection and/or cardiac function and/or respiratory function and/or swallowing function; and
(c) if the subject is diagnosed of suffering from a neurodevelopmental disorder, and optionally of the stage of the disorder, then initiation of a treatment is recommended.

12. The method according to any one of claims 1-11, wherein after the determination of the expression level of the one or more of the miRNA or of the ratio of the said expression levels, the following computer-implemented steps are carried out: said level(s) or ratio(s) are given a value and/or a score, and optionally are computed in a mathematical formula to obtain a computed value; wherein in function of the said level(s), score(s) and or computed value(s), a decision is taken between the options of suffering or not from neurodevelopmental disorder, preferably between suffering or not from Rett syndrome, and/or between the progression of disorder, and/or efficacy of a treatment, and/or decision of recommending initiating a treatment.

13. Use of a kit comprising means for determining the expression level of one of the miRNA of the miR-302/367 cluster and/or of at least one of the miRNA of C14MC, and optionally means for collecting miRNA from EVs, for the diagnosis and/or prognosis of a neurodevelopmental disorder, preferably of Rett syndrome.

14. A kit comprising a means for determining the expression level of at least one of the miRNA of the miR-302/367 cluster and/or of at least one of the miRNAs of C14MC, and optionally means for collecting miRNA from EVs, wherein the means are at least 10% of the reagents in the kit.

15. Use of the expression level in extracellular vesicles (EVs) of at least one or more of a miRNA of the miR-302/367 cluster and/or of at least one or more of a miRNA of the chromosome 14 miRNA cluster, as marker of clinical diagnosis and/or prognosis of a neurodevelopmental disorder and/or for the analysis of the progression of the disorder and/or the monitoring of a treatment for the disorder, preferably for Rett syndrome.
